(19) Europäisches Patentamt / European Patent Office / Office européen des brevets

(11) **EP 2 728 353 B1**

(12) **FASCICULE DE BREVET EUROPEEN**

(45) Date de publication et mention de la délivrance du brevet:
**22.04.2020 Bulletin 2020/17**

(51) Int Cl.:
*G01N 33/24* (2006.01)  *C12N 1/20* (2006.01)
*C05F 11/08* (2006.01)

(21) Numéro de dépôt: **13366005.0**

(22) Date de dépôt: **30.10.2013**

(54) **Procédé d'affectation d'un agro/micro-pedoclimat (AMPC) a une parcelle agricole, et de formation de consortia microbiens**

Verfahren zur Verwendung eines Agro-/Mikro-Pedoklimats (AMPS) auf einer landwirtschaftliche Parzelle und zur Bildung eines mikrobiellen Konsortiums

Method for assigning an agro/micro-pedoclimate (AMPC) to an agricultural plot, and for forming microbial consortia

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.10.2012 FR 1202948**

(43) Date de publication de la demande:
**07.05.2014 Bulletin 2014/19**

(73) Titulaire: **Polyor SARL**
**54000 Nancy (FR)**

(72) Inventeur: **Claude, Pierre-Philippe**
**54000 Nancy (FR)**

(56) Documents cités:
**EP-A1- 2 345 319**

- **Groupement d'intérêt scientifique - sol: "Base de Données Analyse des Terres", , 1 mars 2006 (2006-03-01), XP002699913, Extrait de l'Internet: URL:http://www.gissol.fr/programme/bdat/bdat.php [extrait le 2013-07-02] -& Groupement d'intérêt scientifique Sol: "Base de Données Analyse des Terres", , XP002720206, Extrait de l'Internet: URL:bdat.gissol.fr [extrait le 2014-02-12]**
- **KAMOLCHANOK PANISHKAN ET AL: "Principal Component Analysis for the Characterization in the Application of Some Soil Properties", PROCEEDINGS OF WORLD ACADEMY OF SCIENCE, ENGINEERING AND TECHNOLOGY, 27 mai 2012 (2012-05-27), pages 729-731, XP055102018, Çanakkale ISSN: 1307-6884**
- **L.T. TRAN ET AL: "Application of fuzzy logic-based modeling to improve the performance of the Revised Universal Soil Loss Equation", CATENA, vol. 47, no. 3, 1 mai 2002 (2002-05-01), pages 203-226, XP055069268, ISSN: 0341-8162, DOI: 10.1016/S0341-8162(01)00183-7**
- **ATSUNOBU KADONO ET AL: "Factors controlling potentially mineralizable and recalcitrant soil organic matter in humid Asia", SOIL SCIENCE AND PLANT NUTRITION, vol. 55, no. 2, 1 avril 2009 (2009-04-01), pages 243-251, XP055069267, ISSN: 0038-0768, DOI: 10.1111/j.1747-0765.2008.00355.x**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

**DOMAINE TECHNIQUE DE L'INVENTION**

Microbiologique des sols et pédologie, voire accessoirement la biogéographie des populations bactériennes du sol. L'homme du métier est donc l'agronome, voire le cartographe, ou encore l'agriculteur cherchant à optimiser la biofertilisation de sols arables.

**ÉTAT DE LA TECHNIQUE**

*Le ciblage de* la (ré)introduction de biomasses microbienne biofertilisantes *endogènes* est recherché en agronomie. Ce ciblage contribuer à la plus grande efficacité de biofertilisants non symbiotique (Claude et Fillion 2004). En ce sens, j'ai conçu un mode d'obtention d'azotobactéries à partir des cultures mères de bactéries telluriques (FR2833016), et cela afin de reproduire *in vitro* l'essentiel de ces caractéristiques de ces microhabitats pédologiques. De telles *Azotobacteraceae* n'interagiront qu'avec les *résidusphères* et les *complexes argilo humique* du sol, et ne vont et viennent à travers cette interface (Gaillards *et al.* 1999 et 2003). Leur spécificité est donc "agro-, ou encore micro - pédoclimatique" (AMPC), et non botanique, comme c'est le cas avec des microorganismes en association (*eg. Azospirillum* sp.) et/ou en symbiose (*eg. Rhizobium* sp., mycorhizes) avec la *rhizosphère.* Les biomasses ainsi obtenues et réintroduites dans leurs sols d'origines furent effectivement plus efficaces que leurs consœurs obtenues plus conventionnellement et/ou réintroduites dans un autre type de sol. Cette éventuelle concordance entre AMPC et inoculations (azoto)bactériennes assurant une certaine *endogénicité* des biomasses microbiennes ainsi réintroduites est donc recherchée puisqu'elle dictera en bonne partie l'efficacité *in situ* de tels inocula. J'ai aussi dans un deuxième temps proposé une méthode pour l'obtention de biomasses azotobactérienne particulièrement oligotrophes (EP2314669), et dont elles aussi spécifiquement bien adaptées à la vie dans les sols, voire un AMPC en particulier.

Encore faut-il pouvoir démontrer facilement de telles endogénécités sans pour autant devoir systématiquement prouver leurs origines, leur provenance en sorte, et du coup aussi identifier les biomasses microbiennes les plus efficaces et/ou stables desquelles nous voudrions éventuellement retenir fin d'homologation d'inocula commercialisables entant que tels.

Cela étant, l'utilisation de consortia microbiens et bactériens est donc recherchée (Richaume et al. 2006, Husain et al. 2011, Mendes et al. 2011, Radianingtyas et al. 2003, Jacques et al. 2008). Il est question entre autres des conditions et conséquences de l'entretien *in vitro* d'un consortia de bactéries obtenu directement d'un sol arable (Richaume et al. 2006, de la création de quatre (4) consortia à l'aide de douze (12) souches bactériennes selon leurs cinétiques de croissance (Husain et al. 2011), du fait qu'in situ au niveau des rhizosphères, l'action phytosanitaire des PGPR (*plant growth promoting rhizobacteria*) est nécessairement consortiale (Mendes et al. 2011), ou encore de la la complémentarité des quatre (4) souches (consortia) permet d'attaquer le xénobiotique et ces métabolites (Radianingtyas et al. 2003).

Il existe deux types de consortia ; (i) consortia multi-fonctions et (ii) consortia multi-sites. Or, il ne semble pas y avoir de consensus concernant le nombre et/ou les proportions relatives à respecter de/des diverses souches ou espèces constituant de tels consortia. L'ANSES parle cependant (Anses 2013 ; A17) d'un certain seuil de tolérance à hauteur d'un unité log (10x) applicable au teneurs pondérale et/ou le nombre d'ufc (infra). D'un point de vue industriel et agronomique, il serait avantageux de pouvoir facilement former divers consortia AMPC spécifique, et de déterminer les proportions relatives des souches constituantes.

***Biogéographie et endogénécité des bactéries du sol***

L'aspect clé de cette réintroduction est donc « l'endogénécité » des biomasses bactériennes en question. Cette *endogénécité* implique une *concordance* entre les milieux (sols) d'origines et ciblés. L'*endogénécité* d'une souche/biomasse peut être établit selon la *similarité* entre son milieu d'origine et de réintroduction. S'il était possible de caractériser ce milieu d'origine, et sera ainsi possible de choir pour cette parcelle agricole une biomasse bactérienne spécifique à ce type de milieu tout en respectant le susdit critère d'endogénécité. Or, seuls quelques facteurs clés font déterminer l'essentiel de cette biodiversité ; le pH (eau ou KCl), et la teneur en argile et en carbone organique du sol. En effet, le principal facteur structurant les sols arables est leur teneur en argile, carbone organique ($C_{org}$) et leur pH ; il n'est donc pas surprenant que les bactéries habitant dans des complexes argilo-humique dont la formation est fonction de la teneur en $C_{org}$ et le pH du sol soient mieux adaptées à la vie dans leur sol d'origine qu'autrement.

La biogéographie microbienne, et bactérienne en particulier, des sols fait débat (Curtis *et al.* 2002 ; Fierer et Jackson 2006 ; Fierer *et al.* 2007 ; Cho et Tiedje 2000). L'augmentation de la « distance génétique » plafonne au-delà d'une certaine « distance géographique ». Deux biomasses bactériennes séparées par une distance géographique au-delà d'un certaine seuil - disons et selon les données de Cho et Tiedje 2000 environs 80 km, seront en principe et a *priori* appréciablement distinctes l'une de l'autre ; en dessous de cette distance géographique critique leur distance génétique devrait décroître rapidement et elles peuvent être considérées suffisamment similaire pour être toutes les deux endogènes à leur milieu d'origine commun, et cela dans un rayon donc d'environ 100-80 km.

La répartition de ces *terroirs bactériologiques* est non contiguë. Cette non-contiguïté est problématique lors de l'application de biofertilisations par inoculation de bactéries adaptées à la vie dans les sols en proximité es *résidusphères.* En effet, il est ainsi difficile d'assurer l'*endogénécité* des bactéries ainsi réintroduites en ce référant qu'à des unités cartographiques plus classiques telles que les *pédopaysages* et/ou les *solum* de références très fortement influencés par la susdite biogéographie végétale aérienne. Cette non - contiguïté implique que les UC au sens où l'entend l'homme du métiers et applicable à la végétation aérienne, y compris les cultures agronomiques, ne sont nécessairement applicables au ciblage d'un éventuel inoculum (azoto)bactériens avantageusement AMPC - spécifique.

*Rôle du carbon organique de l'argile et du pH*

La diversité des communautés bactériennes selon l'augmentation de la taille de l'*unité cartographique* (UC) plafonnerait donc éventuellement. Selon Cho et Tiedje 2000, il existerait vraisemblablement une dimension optimale pour de telles UC en termes de diversité bactériologique des sols. Selon Rammet et Tiedje 2007, la variation intra - spécifique des *Burkholderia* est à petite échelle, *i.e.* agrégat des particules de sol, expliqué par les teneurs en argiles et en carbone organique, et à plus grande échelle par le pH du sol. En effet, selon Cho *et* Tiedje 2000, une certaine *auto-corrélation* positive régulant la distribution des *biovars* bactériens sur de courtes distances, soit moins de 100-150 km, devient négative sur de plus grandes distances. En ce sens, selon Ramette et Tiedje 2007 la diversité microbienne est relativement stable sur des distances linéaires de l'ordre de 100-200 m, soit d'au plus quelques hectares.

La fragmentation des micro - habitats du sol (Carson *et al.* 2010, GrunAMPCann et Normand 2000, Vogel *et al.* 2003, Sessitsch *et al.* 2001), notamment en présente de fortes teneurs en argiles et de complexes argilo - humiques développes, est en elle même responsable d'une bonne partie de la diversité génomiques à petite échelle des population bactériennes du sol. L'agrégation des particules du sol, et d'argile plus particulièrement, en segmentant les micro-habitats bactériens, *déconnecte,* en sorte, la biogéographie bactérienne souterraine de la biogéographie végétale aérienne. Ce rôle prépondérant de l'argile explique en partie pourquoi la teneur en argile joue le rôle de variable d'ajustement dans le modèle de la dynamique de la matière organique du sol, RothC (Coleman et Jenkinson 2008).

Fierer et Jackson 2006 et Fierer *et al.* 2007 notèrent une indépendance de la biogéographie des communautés bactériennes du sol aux régimes pluviométriques thermiques aériens, mais aussi l'importante contribution du pH du sol à la diversité phylotypique des bactéries du sol. Il suffit néanmoins de noter que contrairement à la biogéographie des communautés végétales, la biogéographie des communautés bactériennes du sol est *indépendante* des régimes pluviométriques et thermiques à moyennes échelle, *i.e.* bien plus grande que celle de la parcelle et/ou de l'îlot, agricole (Franklin et Mills. 2003, Ramette *et al.* 2007, Van der Gucht 2007, Martiny *et al.* 2011).

**Classification ou *segmentation multivariée***

L'organisation schématique de la SAU (superficie agricole utile) peut ce faire de deux façon ;

> ➢ par ***segmentation*** de la SAU en unités cartographiques (uc) de plus en plus petites selon le nombre de variable pédologiques superposées, avantageusement à l'aide d'un système d'information géographique (SIG) ;

> ➢ par ***rattachement*** d'un solum appartenant à cette SAU à un, ou des, solums de références, a priori au nombre d'une centaine, chaque solum, de référence et à rattacher, ayant été décrit slon une liste exhaustive de variables pédologiques.

Les cartographes / pédologues doivent inscrire une parcelle agricole dans des unités cartographique (UC) plus ou moins imbriquées, et cela à divers niveaux d'agrégations, le plus souvent contiguës de manière à respecter la répartition des roches mères et/ou du climat. Cette contiguïté n'est pas pertinente en ce qui concerne la biogéographie des sols, et donc le ciblage des biofertilisations azotobactériennes. De plus, et malgré la notion de fonctionnement, la répartition de ces unités, bien que répartie sur l'ensemble d'un territoire, demeure essentiellement hiérarchique, et respectent les grandes délimitations climatiques et géologiques. Surtout, il n'est nullement question de correspondances avec les schémas de biogéographie microbienne, et encore moins bactériennes.

Les méthodes de segmentations solutionnent des problèmes de discrimination et de régression en segmentant progressivement le jeu de données matriciel pour obtenir un arbre de décision binaire (Lebart *et al.* 2000). On est généralement en présence d'un tableau, ou jeu, de donnée contenant une variable privilégiée, **y**, à expliquer, en sorte, par les autres variables. Il faut d'une part sélectionner parmi les variables les plus explicatives celles qui sont le plus liées aux variations de la variable continue **y,** et d'autre part de construire une règle de décision permettant d'affecter un nouvel individu à l'une des n - classes plus ou moins homogènes ainsi formées. La segmentation multi - variée est couramment appliquée en pédologie. Elle permet de formuler des unités cartographiques plus ou moins fonctionnelles, à savoir des UTS (*unité topologique des sols*) présentant partout où il est présent la même superposition d'horizons, des UCS (*unité cartographique des sols*) regroupant plusieurs UTS, ou encore des UFS (*unité de fonctionnement des sols) ou unité de paysage* (*alias* catena) dont on connaît l'organisation, voire le fonctionnement.

Pour faire simple, disons que la segmentation hiérarchique caractéristique d'une approche comme celle des pédopaysages implique que l'homme du métier ne vas pas explicitement chercher à créer de nouvelles unités cartographiques en désagrègent des unités cartographiques déjà reconnues comme foncièrement homogènes, mais plutôt à se situer à un quelconque niveau d'agréation au sein d'une même cartographie.

Le rattachement, notamment via la notion *pédopaysage* (Jamagne 2011) plus intégrative d'une certaine écologie des sols, mais surtout de *référentiel pédologique* Afes 2009) qui constitue ici l'état de la technique le plus proche. Il est clairement dit que les couvertures pédologiques, par opposition aux notions de sols et de types de sols, sont fonctions de trois séries de données, (i) dites de constitution, (ii) structurales ou organisation, et (iii) relatives aux dynamiques ou aux fonctionnements des sols. Afes 2009 fait clairement référence à des *espaces typologiques* à N dimensions décrites à l'aide d'un *langage synthétique,* voire aussi *d'ensembles cognats.* Il est aussi question de rattachement d'un *solum* (*i.e.* une tranche verticale d'une couverture pédologique) à une ou plusieurs références, ces dernières à rapprocher encore une fois de la notion d'AMPC. A noter que ce rattachement est un système souple opérable via des méthodes d'analyses multidimensionnelles. Ce type de rattachement à l'aide d'un quelconque RP reconnaît que les couvertures pédologiques sont souvent continues, *i.e.* formés d'unités cartographies (*eg.* UTS, UCS, etc.) contiguës. Il s'agit de classifier des solums en les rattachant à des types de couvertures pédologiques de références. Le référentiel pédologique oblige l'homme de métier, dans la mesure du possible, à assembler les mêmes informations lors de l'observation de la parcelle que lors de la constitution de la base de données pour ledit référentiel. L'existence du référentiel pédologique ne permet pas en soi de raccourcis lors du rattachement d'un solum à une référence typologique. Le référentiel pédologique n'est donc pas un outil de diagnostique et/ou de préconisation pratique au niveau de la parcelle agronomique.

Le référentiel pédologique est foncièrement pédologique et ne considère l'aspect (micro)biologique que comme un facteur de pédogénèse. L'agronome voulant cibler l'inoculation de microorganismes non symbiotiques en parcelles agronomiques n'est pas informé par le référentiel pédologique sur la probable diversité et activité des populations bactériennes endogènes. De plus le référentiel pédologique est vorace en termes d'informations à renseigner. Il faut recueillir un maximum d'information sur le terrain en au laboratoire afin de décrire le solum et son environnement, y compris des analyses, des suivis, des examens de lames fines, etc. Cette démarche de rattachement est donc difficilement applicable commercialement à la parcelle.

Pour revenir enfin à la notion d'endogénécité et biogéographie des populations bactériennes du sol, dans quelle mesure donc y a-t-il concordance entre les sols d'origines et ciblés par la biofertilisation, bactérienne notamment ? A priori, il existe donc deux approches pour établir cette concordance.

1. La superposition d'unités cartographiques de diverses définitions peut ainsi révéler des zones communes et homogènes complexes. En effet, à l'aide de *systèmes d'information géographiques* (GIS) il est possible d'intégrer par superposition des unités cartographiques des divers paramètres pédologiques. Cette superposition permet de repérer au sein de ces unités des régions homogènes en termes des divers paramètres superposés.

➤ Cependant, ces régions homogènes seront nécessairement de sous-ensembles nichés l'un dans l'autre et du coup de plus en plus petits à mesures que l'on inclut dans l'analyse plus en plus de paramètres (Dumanski *et al.* 1993).

2. Le rattachement d'un solum décrit exhaustivement (horizon) à une référence *pédologique* (horizon de référence), cette organisation d'unités cartographiques en diverses sous unités donnant lieu nécessairement à des contiguïtés par association d'ensembles similaires. Ces ACP permettant ainsi de regrouper des uc ou solums selon leurs degrés de similarité ont déjà servis à la classification d'échantillons de sols (Dragovic et Onijia 2006), et l'organisation de zones agricoles selon leurs hydro-morphologies (Carroll *et al.* 2003), potentiels de rendement et/ou aptitudes agroécologiques (Ping *et al.* 2005).

➤ Or, ce regroupement pose problème dans la pratique lorsqu'il faudra situer un quelconque échantillon (une parcelle) dans une telle UC ne comportant, par exemple, qu'un échantillon par 100 à 150 ha.

*Sigles et définitions*

**ACP (analyses par composantes principales)** : Analyse statistique factorielle et multivariée qui transforme des variables liées entre elles (corrélées) en nouvelles variables indépendantes les unes des autres (non corrélées). Ces nouvelles variables, ou composantes principales (CP), permettent de réduire l'information en un nombre de composantes plus limité que le nombre initial de variables.

**CAH (classification ascendante hiérarchique)** : méthode de classification itérative comprenant les étapes suivantes ; i) calcul de la similarité entre les m objets, ii) regroupement des deux objets dont le regroupement minimise un critère d'agrégation donné, iii) recalcule de la similarité entre cette classe et les m-2 autres objets, iv) regroupement des deux objets ou classes d'objets, v) etc. jusqu'à ce que tous les objets soient regroupés.

**Agro/Micro-pédo-climats (AMPC)** : Environnement souterrain agissant directement sur la flore microbienne, bactérienne en particulier ici, composé essentiellement d'agrégats de complexes argilo - humiques, et dont les pH peuvent varier dictant ainsi la composition, l'activité et la résilience desdites communautés microbiennes. Bien qu'imparfait, ce concept permet d'établir la répartition géographique de tels microhabitats non - contiguës.

**Composantes principales 1 et 2 (CP1 et CP2)** : Dans le cadre d'une ACP, les deux première composante principales d'inertie maximale, capable d'englober près voire plus de 50% de la variabilité du « nuage » multidimensionnel formé par la représentation géométrique des *n*-variable.

**Classe (d'argile)** : Segmentation, arbitraire et/ou via un algorithme de classification, de l'ensemble des observations (ici ; 2 607 moyennes cantonales BDAT) en fonction de l'accroissement progressif de leurs teneurs en argiles. C'est à l'intérieure et pour chacune de ces classes que sera établit les trois groupes homogènes I, II et III à titre d'AMPC putatifs.

**Solum** : Tranche verticale d'un sol observable dans une fosse ou une tranchée (Afes 2009).

**Horizon** (pédologique) : volumes plus ou moins parallèles à la surface du terrain dont les dimensions latérales sont largement plus grandes que la dimension verticale (Jamagne 2011)

## DIVULGATION DE L'INVENTION

Problème technique

Le problème technique est donc tripartite ;

i) Le nombre d'unités cartographiques (UC) étant fonction du nombre de variables superposées, très rapidement, la taille moyenne des unités cartographiques va devenir très petite. Le nombre de variables descriptives intégrables pour la formation de telles UC multi - thèmes est donc très limité.

ii) Le rattachement d'une parcelle de terre arable à une telle classe de solum de référence nécessite le renseignement de l'ensemble des variables pédologiques décrivant cette référence, en principe sans exception, ce qui peut devenir très fastidieux. De plus, une telle classe d'observations est en principe homogène, insécable en sorte ; on peut refaire la classification en imposant plus de classes, sans pour autant sous diviser une classe préexistante.

iii) L'état de l'art ne prévoit pas la non-contiguïté d'UC très similaire en termes de composition, de fonctionnement et de structure. Pourtant, de telles UC seraient des cibles identiques lors de la réintroduction de microorganismes endogènes. Or, en termes de biogéographique, la contiguïté des UC est sans importances, la répartition *terroirs bactériologiques* et autres microhabitats étant affranchie de repères géographiques aériens tels que les coordonnées latitudinales et longitudinales, voire la pluviométrie et/ou d'autres moyennes climatiques (*eg.* température, ensoleillement).

Premièrement, une unité cartographique riche en information intégrant bon nombre de variables pédologique sera nécessairement petite, voire unique ; son utilité pour le criblage d'un nombre limité d'inocula bactériens endogènes est donc faible puisqu'il faudrait in démultiplier le nombre de tels inocula propres à autant de (petites) unités cartographiques. Tout cela irait à l'encontre de la vocation d'unité cartographiques intégratifs et donc en nombres limités. On parle ici d'un problème technique lié à la segmentation par superposition d'unité cartographiques ; si l'information est exhaustive, l'étendue de l'inférence géostatistique devient très limitée, *in fine* à une parcelle / îlot.

Deuxièmement, le rattachement d'une parcelle agronomique cible à une de ces unités cartographiques nécessitera la caractérisation exhaustive de ses solum prédominants, soit un exercice plus *pédologique* qu'agronomique, et définitivement trop onéreux pour être économiquement réalisable entant que simple analyse de terre facturée à un agriculteur. On parle ici d'un problème technique lié à la classification (ou rattachement) par proximité euclidienne ; plus l'inférence géostatistique est étendue, plus le complément d'information se doit d'être exhaustif, ce qui rend fastidieux, onéreux et impraticable le rattachement d'un parcelle / îlot à une référence *pédologique* établie simplement afin de pouvoir lui prescrire un inocula (azoto)bactérien endogène.

Troisièmement, la répartition de ces *terroirs bactériologiques* est non contiguë, mais au contraire vraisemblablement réparties à travers une même unité cartographique (UC) au niveau de la parcelle et/ou de l'îlot agricole. Cette non - contiguïté est problématique lors de l'application de biofertilisations par inoculation de bactéries bien adaptées à la vie dans les sols, notamment en proximité es *résidusphères* issues de la dégradation *in situ* de résidus de culture pailleux. Donc, dans le cas de biofertilisations bactériennes, et contrairement la culture de cultivars végétaux, la SAU ne peut pas être simplement ainsi divisée superficiellement en UC ; la préconisation des biovars bactériens ne peut être réalisée aussi simplement. Accessoirement, l'agronome cherchant à assurer l'endogénécité des (azoto)bactéries réintroduites ne veut pas nécessairement ré-isoler chaque fois, pour chaque parcelles ou îlot agricole à traiter, de nouvelles biomasses ; il veut plutôt affecter à chaque parcelle une ou un consortium de souches microbiennes les mieux adaptées à ce type de microhabitats non contigus géographiquement.

Enfin, l'attribution d'un qualitatif pédoclimatique révélateur du fonctionnement des susdits microhabitats à partir d'un simple bulletin d'analyse de terre n'est pas possible à ce jour. Il faut soit procéder à une description exhaustive et en profondeur du sol en question, y compris le repérage de chacun des ses horizons (horizon, solum), taches exigeantes, coûteuses et *in fine* peu adaptées au diagnostique ponctuel / annuel des microhabitats d'une parcelle agronomique, soit cibler des UC trop petites et/ou nécessairement contiguës est donc peut adaptées à la biogéographie des populations microbiennes en sols arables.

Solution technique

Je propose donc de transcrire directement les données pédologiques d'une simple analyse de terre en une valeur d'*agro/micro-pédoclimat* (AMPC), sorte de *terroir bactériologique,* pour lequel un consortium bactérien assurera l'endogénécité des populations (azoto)bactériennes ainsi réintroduites, avantageusement par pulvérisation des résidus de cultures cellulosiques au sol (Claude et Fillon 2004). Les terroirs bactériologiques, ou AMPC, non contigus ainsi définis seront en nombre limité et donc *affectables* à d'importantes SAU, soit avantageusement de l'ordre d'un million d'hectare. De plus, ces AMPC sont issus de la segmentation de classes, elles mêmes obtenues par exemple ici classification ascendante hiérarchique (CAH), et donc en principe, ou du moins par définitions insécables. Une fois segmentée que sur la base des teneurs en carbone ou le pH du sol, ces classes (argileuses) donnent lieu à des AMPC intégrant toujours l'information provenant de l'ensemble des $n$ variables pédologiques.

Cette solution technique est possible du fait que les variables pédologiques teneurs en argile et $C_{organique}$, et pH (eau ou KCl), variables les plus explicatives de la dispersion biogéographique des populations (azoto)bactériennnes du sol, sont généralement les mieux corrélées.avec les deux premières composantes principales issues de l'ACP de jeux de données pédologiques exhaustifs tels que BDAT (Gissol 2006) comportant des milliers observations (moyennes cantonales).

La solution technique comporte deux parties ; (i) l'affectation d'un AMPC à un échantillon de sol, et (ii) la formation de consortia (azoto)bactériens adaptés, voire endogènes, à ces AMPC affectés.

**I.** AFFECTATION DES AMPC

a) Échantillonnage de la parcelle, îlot

Selon les laboratoires les mieux établis, il vaut mieux attendre deux mois après apport d'engrais et quatre mois après apport de matières organiques, amendements calcaires. Il est aussi conseillé de faire le prélèvement hors température froide et hors des périodes de stress hydrique. Idéalement au printemps ou à l'automne. Ne pas prélever dans des endroits inhabituels (bordure de champs, anciennes haies, ancien tas de fumier, mare, zone de passage des animaux...).

b) Analyses physico-chimiques des échantillons

Les analyses physico-chimiques d'échantillons de terre les plus courantes effectuées par bon nombre de laboratoire sont normalisées, à savoir, les teneurs en argile et carbone organique (Co), la capacité d'échange cationique (CEC) et sont degré de saturation (SAT), les teneurs en phosphore, potassium, calcium et magnésium, le pH (eau ou KCl), ainsi que la granulométrie comprenant des mesures de sables et limons, fins et grossiers, et le ratio limons fin et grossier. Il est aussi loisible de déterminer les teneurs en P organique, $pH_{KCl}$, de conductivité électrochimique (EC), voire de certains (oligo)éléments tels que le Fe, l'Al, le Mn, etc., bien que ceux-ci sont parfois peut renseignés par des échantillons de référence (*infra*).

c) Analyses physico-chimiques des échantillons de références → classes argileuses

Dans un premier temps il fallut conditionner la formation de groupes homogènes d'échantillons de sols à leurs teneurs en argiles. Pour ce faire, j'ai effectué une série d'ACP pour l'ensemble des 2605 moyennes cantonales issues de la BDAT (base de données d'analyses de terre ; GISSOL 2006) pour les principales régions agricoles de la France.

Afin de réduire la taille de cette imposante matrice de quelques 18 variables pédologiques variable x 2605 moyennes cantonales, et d'augmenter l'inertie des deux premières composantes principale - CP1 et CP2, j'ai réitéré les susdites ACP plusieurs fois, retirant à chaque fois les variables les moins contributives. In fine, une dizaine de variables pédologiques les plus explicatives furent ainsi retenue ; le pH (eau ou KCl), les teneurs en argile et $C_{organique}$, la CEC et son % de saturation, le logarithme de CaO (pCaO), les teneurs en $K_2O$, ainsi que les fractions granulométriques limons fins et grossiers, et sables grossiers ; l'inertie des CP1 et 2 est de 56%.

d) Attribution de classes argileuses aux échantillons

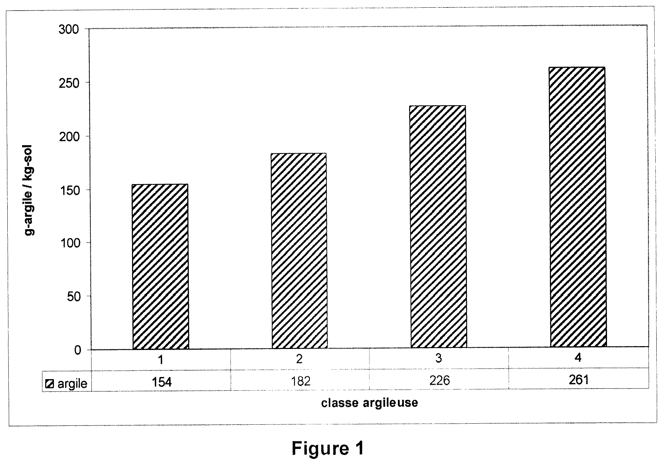

Une classification de l'ensemble des 2605 échantillons de référence par classification ascendante hiérarchique (CAH ; cf. XLStat™ 2010) permit de former des classes argileuses (Figure 1). Il est aussi possible d'utiliser d'autres procédé de classifications tels que l'approches dites "K-Means", etc. ; pour une revue de ces méthodes de classification, voire Lebart et al. 2000.

e) Obtention des composantes principales (CPn)

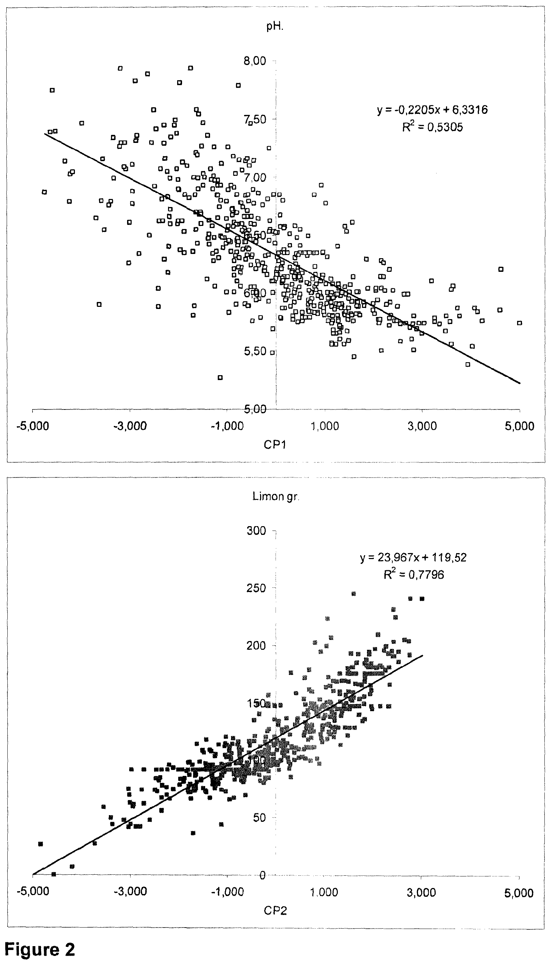

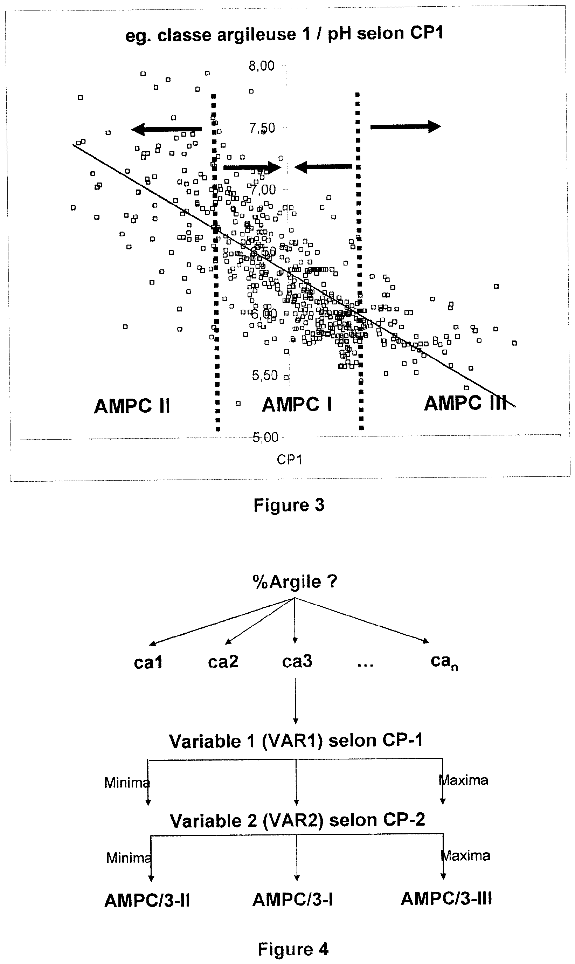

Les deux premières *composantes principales,* CP1 et 2, sont elles corrélées aux variables pH et $C_{organique}$. Il possible de réduire l'interprétation à ces deux seules variables. Graphiquement (Figure 2) ces corrélations entre CP 1 et 2 et, respectivement VAR1 et 2 nous permettent d'utiliser des seuils minimax AMPC spécifiques établissant ainsi trois, I, II et III, AMPC (Figure 3). En effet, pour chacune des ca, la corrélation de variables pédologiques et de *composantes principales* (CPn) permettra d'établir des seuils minima et maxima (minimax) capables de segmenter des groupes homogènes d'observations compris au sein d'une même ca. Des corrélations entre les deux premières composantes principales, CP1 et 2 obtenues par ACP (*analyses en composantes principales*), et deux variables, VAR1 et 2, les plus corrélées sont établies.

Cette approche a déjà été appliquée au traitement de données spectrales de télédétection (Fox et Metla 2005), mais jamais à un ensemble de données pédologique aussi exhaustives. Selon Fox et Metla 2005, les notions de "soil line" (Garey et al. 2005) et de première composante principale (CP1) sont plus ou moins analogues, cette analogie étant intrinsèquement pédo - spécifique. Cette fonction, dite SLED (*Soil Line Euclidian Distance*) est effectivement propre à un sol donné. Ici nous substituerons la notion de sol par celle de classe argileuse. Je propose, au lieu de déplacer le curseur de démarcation (pointillé ; Figure 3) à travers la distance euclidienne déterminée par CP1 puisque c'est bel et bien CP1 qui est pédo - spécifique.

f) Segmentation des classes argileuses → AMPC

La diversité des populations bactériennes du sol étant surtout fonction du pH et de la segmentation physique (spatiale) des microhabitats par les macro-agrégats, il est possible de désigner à partir d'un nombre très limité de classes argileuses un minima d'AMPC obtenues par segmentation, tripartite (Figure 4), de ces classes sur la base de leurs pH, voire leurs teneurs en carbone organique pour les classes à teneur en argile élevées. Les populations bactériennes endogène à ce type d'AMPC le seront nécessairement aussi pour ou dans toutes les parcelles ou îlots possédant les mêmes caractéristiques argileuses, acido-basique et teneurs en carbone organique. A terme, des inocula de bactéries endogènes à chacun de ces AMPC, avantageusement selon FR2833016 et/ou EP2314669, seront préparés.

g) Affectation d'un AMPC à l'échantillon de sol

Cette affectation ce fait simplement à l'aide d'un abaque tel que schématisé à la Figure 4. Un simple bulletin d'analyse de terre comportant usuellement les teneurs en argile et carbone organique (VAR2), ainsi que le pH (eau ou KCl ; VAR1) du sol suffira donc à l'affectation d'un AMPC à la parcelle / îlot agricole d'où provient l'échantillon de terre. L'invention permet ainsi le rattachement d'une parcelle agricole à une références pédologique, ici un AMPC, à partir d'une simple analyse de terre comportant au minimum la teneur en argile, le pH et la teneur en carbone organique. Cette affectation permet le ciblage de biomasses bactériennes non symbiotiques (Claude et Fillion 2004) spécifiquement adaptées à cet AMPC. Elle assure aussi une certaine « endogénécité » des biomasses (azoto)bactériennes ainsi *réintroduites.*

II. FORMATION DES CONSORTIA (AZOTO)BACTÉRIENS

Il est aussi question de consortia, comprenant un minimum de souches (azoto)bactériennes en s'appuyant que sur de simples analyses de terre comprenant au moins les teneurs en argile, en carbone organique et le pH d'un échantillon de terre représentatif de cette parcelle.

i) Analyses physico-chimiques des échantillons de références

Les mêmes analyses physico-chimiques servant à l'affectation des AMPC peuvent servir ici ; il ne sera question cependant que de pH (eau ou KCl), et teneurs en argile et carbone organique du sol.

ii) Comparaison des AMPC affectés et calculés

Il s'agit simplement d'établir une relation empirique, et donc nécessairement imprécise entre les AMPC affectés, pour chacune des observations et/ou échantillons de sol de référence, et les variables pédologiques les plus impliquées dans la biogéographie, la diversité et/ou l'activité in situ des populations (azoto)bactériennes. Cette imprécision donnera lieu à une dispersion qui sera valorisée lors de la formation desdits consortia.

iii) formation des consortia (azoto)bactériens spécifiques aux n x q AMPC affectés

La dispersion des AMPC calculés bornera la gamme d'AMPC calculés d'où proviendront les souches (azoto)bactériennes constituant le consortium le mieux adapté à l'AMPC affecté correspondant.

Concrètement, il s'agit donc d'un procédé de biofertilisation comportant l'affectation d'un *agro/micro - pédoclimat* (AMPC) à une parcelle et/ou un îlot agricole par transcription de variables pédologiques descriptives et d'un échantillon de terre arable en variables agro/micro-pédologiques caractérisé en ce qu'il comprend les étapes suivantes ;

- l'échantillonnage de la parcelle et/ou l'îlot agricole pour fin d'analyse physico-chimique ;
- l'analyse physico-chimique dudit échantillon de terre générant des variables pédologiques afin de lui associer une description physico-chimique comprenant au moins les variables, teneur en argile et en carbone organique, pH eau ou KCl, voire la CEC et/ou la granulométrie ;
- l'analyse physico-chimique d'un ensemble d'échantillons de terre afin d'effectuer une classification des ces échantillons sur la base de leurs teneurs en argile, créant ainsi une gamme de n classes argileuses ;
- l'attribution d'une classe argileuse à cet échantillon de terre, et donc par inférence à ladite parcelle et/ou îlot agricoles des quels il provient ;
- au sein d'une même classe argileuse, l'analyse physico-chimique préalable d'un ensemble d'échantillons de terre de référence afin d'extraire de cet ensemble une série de composantes principales (CPn) pouvant être mise en relation avec l'un ou l'autre des susdites variables pédologiques ;
- la segmentation des dites classes argileuses en un nombre q agro/micro - pédoclimat (AMPC) à l'aide de seuils minimax obtenues par mise en relation desdites variables pédologiques et des deux premières CPn, CP1 et CP2, les plus corrélées
- l'affectation d'un AMPC à l'échantillon de terre par, (i) comparaison de sa teneur en argile afin de le situer dans une classe argileuse particulière, et (ii) par comparaison de son pH ou sa teneur en carbone organique afin de déterminer leur position par rapports au susdits seuils minimax.

La formation des classes argileuses ce fait à l'aide d'une classification ascendantes hiérarchique d'un ensemble préalable d'échantillons de terre ; le nombre de classes argileuses est compris entre 2 et 20, plus particulièrement entre 3 et 16, et avantageusement ici de 4. Les CPn sont eux obtenues par *analyse* multivariée, y compris ici avantageusement par *composantes principale* (ACP). Les desdites CPn sont mises en relation par régressions linéaires [$VAR_{1/2}$ : $CP_{1/2}$], où $VAR_{1/2}$ sont les deux variables pédologiques les plus corrélées aux deux premières *composantes principales,* $CP_{1/2}$, respectivement, issues de ladite analyse multivariée.

Les *n* classes argileuses sont ensuite segmentées en *q* AMPC à l'aide de *seuils minimax* applicables à chacune des classes argileuses, cette segmentation donnant une un nombre (*n x q*) d'AMPC attribuables. Pour ce faire, il est avantageux que les que les seuils minimax ($MiniMax_{1/2}$) applicables à chacune des *n* - classes d'observations soient déterminés par la formule suivante ;

$$[MinMax_{1/2} = \text{moyenne de } VAR_{1/2} +/- (\text{pente de la régression linéaire } [VAR_{1/2} : CP_{1/2}] \times R^2_{1/2})],$$

lorsque $VAR_{1/2}$ sont les deux variables pédologiques, hormis celle ayant servie à la classification en groupes homogène suite à l'ACP, les plus corrélées avec $CP_{1/2}$, *i.e.* les coordonnées des deux premières composantes principales des *n* - ACP, respectivement, et $R^2_{1/2}$ les deux coefficients de déterminations desdites régression linéaires [$VAR_{1/2}$ : $CP_{1/2}$], respectivement.

Il est aussi loisible et avantageux que l'application des seuils minimax est conditionnée par la co-*inertie* entre les variables VAR1 et 2 permettant d'établir des *intervalles floues* (if) déterminées comme suit ;

$$if = [(\text{coordonnée } CP1 = R^2 \mid [VAR_1 : CP_1]) : (\text{coordonnée } CP2 = R^2 \mid [VAR_2 : CP_2])],$$

ces intervalles de confiance étant ainsi applicables directement aux régressions $[VAR_{1/2} : CP_{1/2}]$ de manière à identifier la plage de valeurs que peuvent prendre VAR1 et 2 observées pour les susdits échantillons de terre arable. *In fine,* il sera ainsi possible qu'au moins deux échantillon de terre géographiquement distants d'environs 80 kms ou plus se voient attribuer un même AMPC.

Il est aussi question d'un autre procédé de biofertilisation complémentaire, cette fois-ci pour la formation de consortia (azoto)bactériens par comparaison des AMPC affectés selon une quelconque des revendications précédentes et des AMPC calculés à l'aide d'une fonction empirique comportant ;

> une prise d'échantillon de terre de la parcelle et/ou de l'îlot agricole afin d'obtenir une analyses physico - chimique ;

> une analyse physicochimique de cette échantillon de terre afin de lui affecter une valeurs d'AMPC;

> l'affectation d'un AMPC à cette échantillon de terre ;

> une analyse physicochimique d'un ensemble d'échantillons de terre de référence afin d'établir une relation multivariée empirique entre les susdites valeurs d'AMPC affectés et les valeurs des variables pédologiques les plus impliquées dans la détermination des niveaux d'activité et/ou de diversité des populations (azoto)bactériennes du sol ;

> comparaison graphique et/ou mathématique entre ces AMPC calculés et affectés afin d'obtenir une appréciation de la dispersion des AMPC calculés alentour des AMPC affectés ;

> formation, à titre de consortia, pour chacun des AMPC affectés de gammes de souches (azoto)bactériens provenant d'AMPC affectés compris dans cette dispersion d'AMPC calculés alentour de chacun de ces valeurs d'AMPC affectés.

La relation multivariée empirique est une régression linéaire ou non - linéaire, tandis que les variables pédologiques les plus impliquées la détermination du niveau d'activité et/ou de diversité des population (azoto)bactériennes du sol sont choisi parmi un groupe comprenant le pH, les teneurs en argile et en carbone organique, voire le CEC et/ou la granulométrie. Les valeurs des AMPC ainsi calculés pour chacune des valeurs AMPC affectés sont distribuées normalement, et avantageusement arrondies à des nombres entiers. Du coup, la fréquence et/ou la densité de probabilité associé à chacune des valeurs des AMPC calculés ainsi arrondies détermine la proportion relative dans le consortia des souches (azoto)bactériennes provenant de AMPC affectés.

*Avantages apportés et activité inventive*

Attribuer à une parcelle une classe *argileuse* au lieu d'une plus usuelle classe *texturale,* et cela à partir d'une simple analyse de terre, est contre intuitif. En effet, l'homme de métier considérera que la classe texturale basée sur la granulométrie du sol et plus informative qu'une telle classe argileuse a priori plus réductrice. De plus, le fait de *segmenter* par la suite ces classes argileuses est inhabituel ; cette segmentation, a *posteriori* en sorte, est contraire aux habitudes de l'homme du métier, soit ici le pédologue - agronome, qui cherchera à créer des unités cartographiques foncièrement insécables. Pourtant, c'est bel et bien la segmentation de ces classes argileuses, le plus souvent sur la base du pH du sol, qui permet ici de transcrire un simple bulletin d'analyse de terre comportant que des données pédologiques, en préconisations microbiologiques, et cela sans avoir à mener de coûteuses déterminations de la biomasse ou de l'activité microbienne *in situ,* ou encore de rattacher la parcelle à une quelconque référence typologique.

## DESCRIPTION DES DESSINS ET FIGURES

**Figure 1** : Les quatre groupes homogènes, ou classes, argileuses obtenues par classification ascendante hiérarchique (CAH) des 2605 observations (moyennes cantonales) des variables pédologiques retenues après réduction de la matrice à 10 variables pédologiques par élimination des variables pédologiques les moins explicatives (supra). Pour chacune de ces classes argileuses, les moyennes correspondent aux barycentres, et progressent régulièrement facilitant ainsi l'utilisation de statistiques descriptives pour fins d'attribution d'un échantillon à une telle classe argileuse.

**Figure 2** : Pour la classe argileuse no. 1 (barycentre 154 g/kg), les corrélations entre les deux variables pédologiques les mieux corrélées avec les deux premières composantes principales (CP1 et 2) issues d'une analyse par composante principale (ACP) des 561 observations (moyennes cantonales) des neuf (9) variables (Tableau 1). Ces corrélations sont ainsi établies ainsi pour chacune des 4 (quatre) classes argileuses (Figure 1). L'argile servant ici à classifier les 4 grands groupes homogènes, cette variable n'est pas incluse dans ces ACP.

**Figure 3 :** Exemple de segmentation d'un groupe homogène (classe) à l'aide de la corrélation entre la variables pédologique, VAR1, la mieux corrélée avec la première composante principale, CP1 (Figure 2). Les seuils minimax (MiniMax1/2) applicables à chacune des n - classes d'observations sont déterminés par la formule [MinMax1/2 = moyenne de VAR1/2 +/- (pente de la régression linéaire [$VAR_{1/2}$ : CP1/2] x R21/2)], lorsque VAR1/2 sont les deux variables pédologiques, hormis celle ayant servie à la classification en groupes homogène suite à l'ACP initiale, les plus corrélées avec $CP_{1/2}$, i.e. les coordonnées des deux premières composantes principales des n - ACP, respectivement, et R21/2 les deux coefficients de déterminations desdites régression linéaires [VAR1/2 : CP1/2], respectivement. Plus $R^2$ est important, plus les seuils minimax seront éloignés et plus l'AMPC central (I) sera probable (fréquent) au dépends des AMPC II et III.

**Figure 4 :** Abaque décrivant la formation des AMPC (Tableau 2) par segmentation des n - classes argileuses selon l'une et l'autre des deux variables pédologiques (VAR1, 2) les mieux corrélées aux deux premières composantes principales (CP1, 2). Des seuils minimax permettent ainsi l'attribution de l'échantillon appartenant à une classe argileuse particulière à l'un des trois sous groupes I, II ou III, alias AMPC. L'attribution de l'AMPC selon la première variable corrélée avec CP1 (VAR1) devrait, en principe, correspond à l'AMPC attribué selon la deuxième variable corrélée avec CP2 (VAR2).

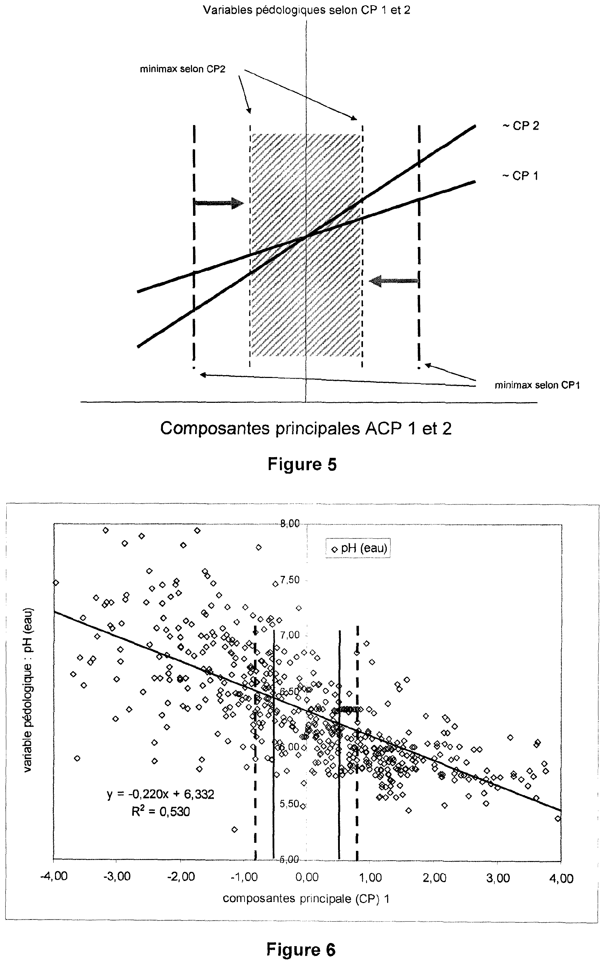

**Figure 5** : Schéma de formation des intervalles floues (*if*; Tableau 3) de l'intersection entre les deux zones minimax selon CP1 (VAR1) et CP2 (VAR2), ainsi que la correction (flèches) des seuils minimax CP1 en fonction des seuils minimax CP2. A noter que la zone minimax selon CP2 est ici un sous-ensemble de la zone minimax selon CP1; il existe donc un *intervalle floue* (if) entre ces deux zones.

**Figure** 6 : Exemple d'application du schéma à la Figure 5 permettant la segmentation de la classe argileuse no. 1 (barycentre 154 g/kg) à l'aide d'intervalles floues (if). La formation des seuils minimax est maintenant conditionnée par la co-inertie entre les variables pédologiques VAR1 et 2 établissant des intervalles floues (if) selon l'équation ;

$$if = (\text{coordonnée CP1} = R^2| \text{[VAR1 : CP1]}) : (\text{coordonnée CP2} = R^2| \text{[VAR2 : CP2]}),$$

ces intervalles de confiance étant ainsi applicables directement aux régressions [VAR1/2 : CP1/2] (Figures 2 et 3) de manière à identifier la plage (intervalle) de valeurs que peuvent prendre VAR1 et 2 lors de l'attribution de l'un des trois (I, II ou III) AMPC à un échantillon de terre appartenant à cette classe argileuse (Figure 4, Tableau 3). Ces if sont ainsi conditionnés par la co-inertie de VAR1 et 2.

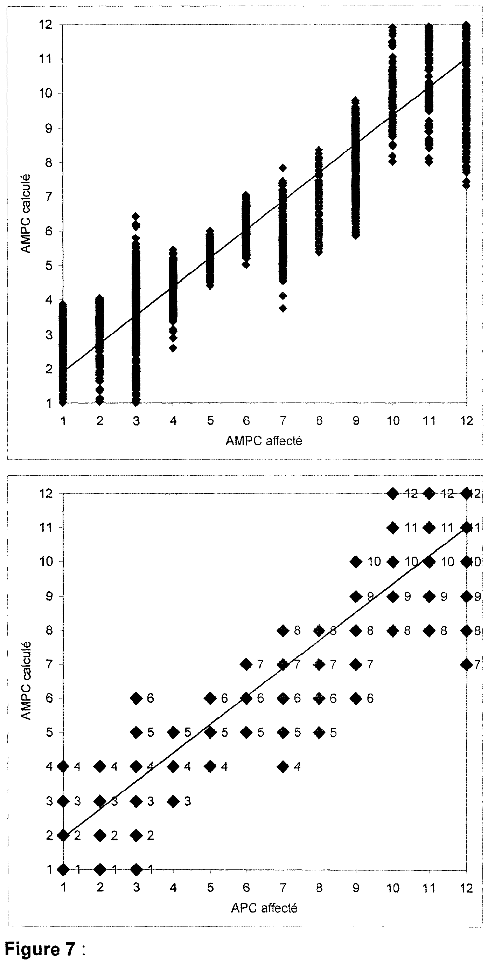

**Figure 7** : Corrélation entre les AMPC *calculés* selon l'équation (1) et leurs AMPC *affectés* correspondant (figure du haut). Une fois arrondies (figure du bas), les valeurs des AMPC révèlent le nombre et le numéro de l'AMPC d'origine des éventuelles souches (azoto)bactérienne constituant les consortia les mieux adaptés aux AMPC affectés correspondant ; les compositions de ces douze (12) consortia en % de chacune des souches d'origine sont rapportées au Tableau 4.

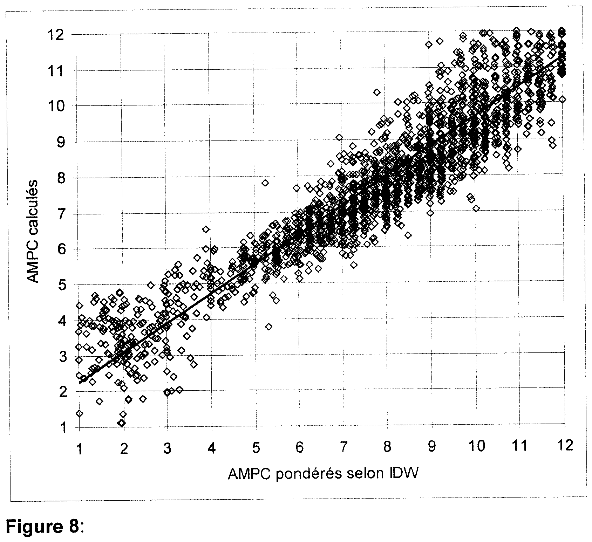

**Figure 8 :** Corrélation entre les AMPC *calculés* et les AMPC affectés mais dont les valeurs sont pondérées par l'indice selon l'équation (2), dit "inverse distance weighting", ou IDW.

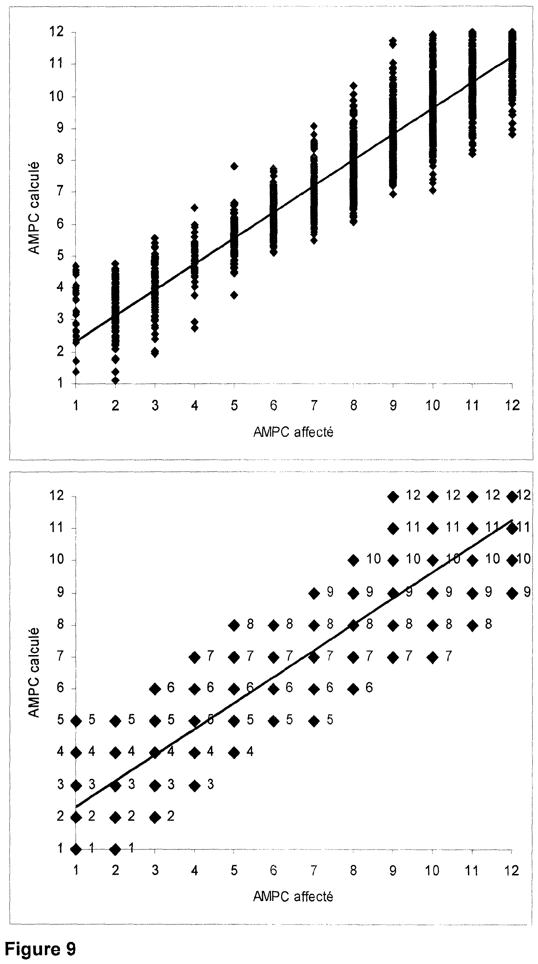

**Figure 9** : *idem* à la Figure 7, mais avec des AMPC calculés selon l'équation (4) une fois les valeurs des AMPC affectés pondérés géostatistiquement selon IDW (Figure 8) arrondies. A la figure du haut, les valeurs AMPC *calculés* non arrondies, et en bas ces mêmes valeurs arrondies ; les compositions de ces douze (12) consortia en % de chacune des souches d'origine sont rapportées au Tableau 5.

**RÉALISATION DE L'INVENTION**

***Schéma de réalisation*** *: désignation des AMPC)*

a) Échantillonnage de la parcelle

Sur toute la profondeur du labour, mais que de 0 à 10 cm sous prairie permanente, en éliminant les débris de surface. Pour les vergers, à 2 profondeurs différentes, soit en général 0 à 20 cm et de 20 à 50 cm. Il est généralement recommandé d'effectuer au moins une quinzaine de prélèvements, de les mélanger dans un seau, et de mettre 500 g à 1 Kg dans un sachet ou flacon. Il recommandable d'envoyer de l'échantillon en carton isotherme avec bloc réfrigérant pré congelé.

Il est aussi possible d'utiliser pour l'échantillonnage un dispositif (kit) d'échantillonnage (demande FR 12/01990) spécialement adapté comprenant Filtre macro-microporeux constitué d'un feuilleté multi-plis semi-rigide. Les différentes couches, sous la forme d'opercules rétractibles, peuvent être retirées systématiquement, de manière à apporte aseptiquement à l'échantillon de l'eau et/ou une solution nutritive sans avoir à manipuler directement l'échantillon et/ou rompre le lien aseptique qui le protège. Ce mode de préparation de l'échantillon est particulièrement utile si on veut orienter le développement de la flore (azoto)bactérienne avant l'isolement de souches AMPC spécifiques.

b) Analyses physico-chimiques (normalisées) des échantillons proposés ;

- **Argile** : *proportion de particules inférieures à 2 $\mu$ après sédimentation et destruction des agrégats, mais sans décarbonatation (norme X 31-107 ; g / kg-du sol ; moyenne BDAT - 203).*

- **$C_{organique}$** : *dosé après minéralisation au dichromate de potassium (norme NF X31-109 ; g-C /kg-sol ; moyenne BDAT = 18.3).*

- **CEC** (Metsun, Rhieum) : *déterminée par échange avec l'acétate d'ammonium à pH 7 (norme NF X31-130 ; $cmol^+$/kg de terre fine ; moyenne BDAT = 11.60). i.e. Quantité de cations qu'un échantillon peut retenir sur con complexe absorbant à pH 7.*

- **Phosphore** (Joret, Dyer, Olson) : *extrait par solubilisation dans une solution d'oxalate d'ammonium 0,1 $mol.l^{-1}$ (norme NF X31-161 ; mg $P_2O_5.kg^{-1}$, moyenne BDAT = 224).*

- **Potassium** : extrait par une solution d'acétate d'ammonium 1 mol.$1^{-1}$ à pH 7, puis dosé par spectrométrie (norme NF X31-108 ; mg $K_2O.kg^{-1}$, moyenne BDAT = 261).

- **Calcium** *(calcaire (ou carbonate) tota(aux)l) : déterminée par dosage volumétrique du $CO_2$ dégagé par acide fort à température ambiante (norme NF ISO 10693 ; g/kg-sol ; moyenne BDAT = 105).*

- **Magnésium** : extrait par une solution d'acétate d'ammonium 1 mol.$l^{-1}$ à pH 7, puis dosé par spectrométrie (norme NF X31-108 ; mg MgO / kg-sol ; moyenne BDAT = 145).

- **$pH_{eau}$** : déterminé par mesure électrométrique d'une solution échantillon/eau selon un rapport massique 1/5 (norme NF ISO 10390 ; moyenne BDAT = 6.75).

- **Sables** : *proportion de particules comprises entre 50 et 2000 $\mu$m après tamisage et destruction des agrégats, mais sans décarbonatation (norme X 31-107 ; g / kg-sol ; moyenne BDAT = 310).*

- **Limons fins et LF/LG** : *rapport entre limons fins (de 2 à 20 $\mu$) et grossiers (entre 20 et 50 $\mu$) selon une méthode de sédimentation (norme X31-307; moyenne BDAT = 1,33).*

- **Taux de saturation** : rapport entre la quantité de cations échangeables pouvant être retenue par la CEC et la somme des cations échangeables Ca++, Mg++, K+ et Na+ ; moyenne BDAT = 143.

c) Analyses physico-chimiques des échantillons de références → classes argileuses

Pour réaliser l'invention il faut avantageusement un jeu de données pédologiques comprenant au moins les variables pH du sol (eau ou KCl), teneurs en carbone ou matière organique (Co ; g-C / kg-sol) et argile (g-argile / kg-sol), la capacité d'échange cationique (CEC ; cmol$^+$ / kg-sol (fin) et son degré de saturation cationique (SAT = %(Ca$^{2+}$ + Mg$^{2+}$ + K$^+$ + Na$^+$) / CEC), voire les teneurs en CaO, MgO et K$_2$O (mg / kg-sol), les teneurs en Na$_2$O étant généralement assez faibles, du moins en Europe de l'ouest. Il est aussi loisible d'inclure en complément les teneurs de sables (fins et/ou grossiers ; g / kg-sol) et/ou limons (fins et/ou grossiers ; g / kg-sol).

Les jeux de données doivent être les plus complets et exhaustifs possible ; voire *supra* la section I. b) Affectation des AMPC - analyses physico-chimiques des échantillons. Il existe pour la France une base de données comportant suffisamment d'observations en ce sens ; la *Base de données d'analyses de terre* (BDAT ; GISSOL 2006). Or, bon nombre de variables ne sont pas renseignées pour l'ensemble des cantons. Puisque trop de données manquantes pouvant fausser l'analyse, les cantons non renseignés étant omis des tableaux BDAT, il faut donc aligner (apparier) les différentes valeurs pour chaque variable pédologique. Étant donnée des milliers de moyennes cantonales à vérifier une par une pour chacune des variables, cela peut être assez fastidieux. *In fine,* j'ai retenue ici 2605 moyennes cantonales suffisamment renseignées pour l'ensembles des susdites variables pédologiques.

Il est aussi possible d'inclure des variables synthétiques construites à partir de variables connues, veillant que ces variables synthétiques ne soient pas excessivement corrélées entre elles et avec les variables connues. Par exemple, le phosphore organique peut être estimé grossièrement à partir des données pH, Al, et Fe (Turner et al. 2003), ou encore de conductivité électrique à partir de pH eau et KCl. A ce stade, j'ai trouvé que de telles variables synthétiques sont trop corrélées avec les autres variables. De plus (*supra*) ces variables comportent trop peu de moyennes cantonales, et les données manquantes à générer trop nombreuses. A suivre.

Une fois cette matrice de m observations x p variables établie, une première série d'ACP afin de réduire le nombre de variables pédologiques. Ces ACP peuvent sont réalisées de manière à ce que l'orthogonalité des CP1 et 2 comprend l'ensemble des variables pédologiques retenues. Il est facile de réaliser cette réduction en éliminant par réitération de l'ACP les variables pédologiques les plus corrélées avec la CP la moins importante, et ainsi de suite à chaque réitération, jusqu'à ce que toutes les variables retenues soient corrélées avec CP1 ou 2. *In fine,* j'ai ainsi retenu dix (10) variables pédologiques (*infra,* Tableau 1).

d) Attribution de classes argileuses aux échantillons

Avant d'attribuer un AMPC à un échantillon de terre, il faut lui attribuer une classe argileuse. Or, les classifications ascendantes hiérarchique (CAH) ne fournissent que des barycentres (Tableau 1) plus ou moins distants les uns des autres ;

**Tableau 1** : Barycentres des variables pour les quatre (4) classes argileuse

| Classe | n-obs | argile | Co | pH-eau | CEC | pCaO | K2O | saturation | sables gr, | limons fins | limons gr, |
|---|---|---|---|---|---|---|---|---|---|---|---|
| 1 | 561 | 154 | 19,42 | 6,34 | 10,99 | 0,46 | 218 | 141 | 395 | 180 | 120 |
| 2 | 483 | 182 | 15,86 | 6,97 | 11,39 | 1,37 | 247 | 179 | 102 | 242 | 360 |
| 3 | 1150 | 226 | 18,83 | 6,82 | 13,08 | 1,27 | 262 | 162 | 197 | 253 | 181 |
| 4 | 413 | 261 | 15,19 | 7,86 | 13,63 | 2,23 | 321 | 386 | 195 | 243 | 165 |

Le barycentre lui même peut servir de borne, ce qui est imparfait puisse qu'il s'agit bien d'un centre et non d'une limite ; voir infra f). Il est aussi possible d'utiliser des statistiques descriptives propres à ces classes, par exemple les bornes inférieures et supérieures (95%), bien que généralement trop étroites. Il est aussi possible de simplement appliquer l'écartype/2 de part et d'autre la valeurs moyenne (154, 182, etc.) ; dans ce cas là, c'est le contraire, et les classes ainsi délimitées ce chevauchent. Or, il s'avère que l'*écart absolue,* moyen ou médian, est souvent le mieux adapté à la délimitation de classes mutuellement exclusives (*i.e.* sans chevauchements). Il est donc recommandable d'adopter cette statistique / 2 de part et d'autre du barycentre (*infra,* Tableau 2).

e) Obtention des composantes principales (CPn)

La formation de classes *segmentables* par après selon les seuils minimax (*infra*) doit respecter soit la VAR la plus corrélée avec CP1 de l'ACP, soit dictée par son rôle prépondérant sur les *microhabitats* ; la teneur en argile joue ici ce rôle. Par exemple, ici quatre classes argileuses ont été retenues ; 12 (4 x 3) AMPC seront donc attribuables. Pour chacune de ces classes argileuses, une ACP est effectuée, sans la variable argile comme de raison, afin d'obtenir une série de composantes principales (CPn) propres à chacune de ces classes argileuses, et plus ou moins corrélées avec certaines des variables pédologiques (Figure 2).

**Tableau 2** : Découpage des quatre (4) classes argileuses en 3 x 4 = 12 AMPC selon les seuils minimax calculés à partir des régressions linéaires [$CP_{1/2}$ : $VAR_{1/2}$].

| g-argile par kg-sol | | | | sMinimax VAR1 | | sMinimax VAR2 | |
|---|---|---|---|---|---|---|---|
| (barycentre) +/- écartype/2 | AMPC | VARiable 1 | VARiable 2 | minimum | maximum | minimum | maximum |
| 140 - (154) - 170 | 1 | | | 6,21 | na | 101 | na |
| | 2 | pH | Limons gr. | 6,21 | 6,45 | 101 | 139 |
| | 3 | | | na | 6,45 | na | 139 |
| 171 - (182) - 195 | 4 | | | 6,68 | na | 10,58 | na |
| | 5 | pH | CEC | 6,68 | 7,26 | 10,58 | 12,18 |
| | 6 | | | na | 7,26 | na | 12,18 |
| 196 - (226) - 253 | 7 | | | 6,58 | na | 16,9 | na |
| | 8 | pH | Co | 6,58 | 7,05 | 16,9 | 20,9 |
| | 9 | | | na | 7,05 | na | 20,9 |
| 254 - (261) - 500 | 10 | | | 13,5 | na | 7,67 | na |
| | 11 | Co | pH | 13,5 | 16,9 | 7,67 | 8,05 |
| | 12 | | | na | 16,9 | na | 8,05 |

f) Segmentation des classes argileuses en AMPC

Pour segmenter chacune des classes argileuses, ici et à titre d'exemple en trois (3) AMPC, deux (2) seuils minimax sont requis. Pour ce faire, les coefficients de détermination ($R^2$) régressions linéaires des variables les mieux corrélées avec CP1 et 2 pour chacune desdites classes argileuses sont multipliés par la moyenne de la variable (i.e. lorsque $CP_{1/2}$ = 0,00 ; Figure 3). Deux valeurs de variables pédologiques sont ainsi calculées fonction donc de leurs degrés de corrélation avec $CP_{1/2}$ (eg. Figure 2). Pour borner les AMPC il s'agit de tabuler les susdits seuils minimax applicables aux échantillons de terre ; ici et par exemple une série de douze (12) AMPC établis à partir des 4 classes argileuses (Tableau 2). Voir à la Figure 4, un abaque schématisant ce processus d'affectation aux échantillons de sols appartenant à une même classe argileuse.

Or, dans les faits, ces seuils minimax et les démarcations qu'ils impliquent, sont graduels, et il existe nécessairement des zones de transitions se chevauchant ; de simples règles d'affectation abruptes sont souvent inappropriées puisqu'un certain nombre d'échantillons se trouvera immanquablement compris dans ces zones « floues ». Or, en pédologie ce *floue* peut être pris en compte mathématiquement via la *logique floue* (Zadeh 1965, McBartney et Odeh 1997, Zhu *et al.* 2001, Tran *et al.* 2001). Cela permet d'éviter une incohérence entre la désignation des AMPC selon VAR1 et 2 (*infra*). Ces règles permettent d'établir un intervalle, une gamme en sorte, de valeurs que peuvent prendre $VAR_{1/2}$ sans rompre le cadre du présent schéma d'attribution. J'ai rapporté à la Figure 5 une représentation schématique de cette application de la logique floue, tandis qu'à la Figure 6 apparaît la formation desdites intervalles floues (if) pour la première classe argileuse (154 g/kg). Ainsi, la première variable pédologique, VAR1, qu'est ici pH du sol peut être compris entre une plage de valeurs de manière à assouplir l'attribution d'un AMPC toute en respectant l'essentiel des règles d'attribution ; *idem* pour la deuxième variable pédologique, VAR2, qu'est la teneur en limons grossiers, et ainsi de suite pour les trois autres classes argileuses ; ces intervalles flous sont rapportés au Tableau 3 ;

**Tableau 3** : *Intervalles floues* (if) permettant d'affecter un des douze AMPC à un échantillon

| Classe argileuse | AMPC | VAR1 | if Mini | if Maxi | VAR2 | if Mini | if Maxi |
|---|---|---|---|---|---|---|---|
| | 1 | | 6,16 : 6,21 | | | 101 : 106 | |
| 154 | 2 | pH | 6,16 : 6,21 | 6,45 : 6,51 | Limons gr. | 101 : 106 | 134 : 139 |
| | 3 | | | 6,45 : 6,51 | | | 134 : 139 |
| | 4 | | 6,68 : 6,74 | | | 10,4 : 10,6 | |
| 182 | 5 | pH | 6,68 : 6,74 | 7,16 : 7,26 | CEC | 10,4 : 10,6 | 12,2 : 12,4 |
| | 6 | | | 7,16 : 7,26 | | | 12,2 : 12,4 |
| | 7 | | 6,58 : 6,66 | | | 16,0 : 16,8 | |
| 226 | 8 | pH | 6,58 : 6,66 | 6,99 : 7,05 | Co | 16,0 : 16,8 | 20,9 : 21,7 |
| | 9 | | | 6.99 : 7,05 | | | 20,9 : 21,7 |
| | 10 | | 13,2 : 13,5 | | | 7,67 : 7,70 | |
| 261 | 11 | Co | 13;2 : 13,5 | 16,9 : 17,1 | pH | 7,67 : 7,70 | 8,02 : 8,05 |
| | 12 | | | 16,9 : 17,1 | | | 8,02 : 8,05 |

Cette correction aura pour effet réduire les seuils maxima et d'augmenter les seuils minima utilisés précédemment pour l'attribution d'un AMPC. Pour ce faire, je me suis inspiré ici des travaux de Tran *et al.* 2001 en utilisant une ordination des CP1 et 2 et des variables pédologiques VAR1 et 2 qui leur sont le mieux corrélées (Figures 6). Pour VAR1 les minima seront donc augmentés, et les maxima réduits au profit d'un recentrage sur l'*inertie* caractéristique de VAR2.

g) Affectation d'un AMPC à l'échantillon de sol

Cet affectation peut donc maintenant ce faire qu'à l'aide d'un simple bulletin d'analyse de terre comprenant des valeurs pour les teneurs en argiles et $C_{organique}$ du sol, et le pH (eau ou KCl).

***Schéma de réalisation : *** *formation des consortia (azoto)bactériens*

i) Analyses physico-chimiques préalables des échantillons de références

Pour la formation des consortia, il suffit de considérer seulement les teneurs en carbone organique et en argile, et le pH, eau ou KCl, soit un sous ensemble du jeux de données ayant servi à l'établissement des classes argileuses et des CPn lors de l'affectation des AMPC (*supra*).

ii) Comparaison des AMPC affectés et calculés

Prenons pour exemple le cas des quatre (4) classes argileuses, et des 12 AMPC affectables. Il est ainsi possible de *calculer* une valeurs d'AMPC à l'aide d'une régression linéaire multivariée des 2605 teneurs en argile et $C_{organique}$, et le $pH_{eau}$ comme variables explicatives ;

$$AMPC_{calculé} = 0,0419[argile] - 0,0148[C_{organique}] + 1,0706[pH_{eau}] - 9,612 ; R^2 \ 83\% \qquad (1)$$

Cette régression linéaire permet de calculer directement une valeur continue d'AMPC entre 0,00 et 12,00. Il est possible rapporter graphiquement ces valeurs continues par rapport aux AMPC affectés de 1 à 12, affectés par la classe d'argile et le pH de l'échantillon de sol (Figure 7, haut). A noter qu'il existe ainsi, pour chacun des AMPC désignée une gamme d'AMPC calculés ; cette gamme (Figure 7, bas) comprend en principe les consortia de souches AMPC spécifiques applicables pratiquement à chacune des parcelles pour lesquelles nous aurons attribué un AMPC unique. Pour rendre plus lisible ce schéma, j'ai arrondi les valeurs d'AMPC calculés (Figure 7, bas), chaque valeur précisant ainsi la souches AMPC spécifique devant faire partit du consortium.

iii) Formation des consortia (azoto)bactériens spécifiques aux n x q AMPC

La valorisation de la dispersion des AMPC calculés alentour des AMPC affectés selon la relation empirique **(1)** permettra la formation de consortia aux proportions (%) des souches provenant des divers AMPC affectés tel que proposé ici au Tableau 4 ;

Tableau 4 : Composition des consortia bactériens endogène adaptées aux AMPC affectés (v1).

| AMPC | | Calculés ➜ % des souches membre du consortium adapté à l'AMPC affecté selon leur origine | | | | | | | | | | | |
|---|---|---|---|---|---|---|---|---|---|---|---|---|---|
| | | 1 | 2 | 3 | 4 | 5 | 6 | 7 | 8 | 9 | 10 | 11 | 12 |
| Affectés | 1 | 10% | 00% | 37% | 53% | | | | | | | | |
| | 2 | 6% | 00% | 35% | 58% | | | | | | | | |
| | 3 | 4% | 00% | 25% | 00% | 59% | 00% | 12% | | | | | |
| | 4 | | 2% | 36% | 50% | 11% | | | | | | | |
| | 5 | | | 00% | 49% | 51% | | | | | | | |
| | 6 | | | | 8% | 56% | 36% | | | | | | |
| | 7 | | | 1% | 10% | 49% | 31% | 10% | | | | | |
| | 8 | | | | 36% | 27% | 37% | | | | | | |
| | 9 | | | | 1% | 15% | 35% | 36% | 13% | | | | |
| | 10 | | | | | | | | 21% | 50% | 29% | | |
| | 11 | | | | | | | | 13% | 45% | 28% | 14% | |
| | 12 | | | | | | | | 5% | 00% | 00% | 95% | |

Sur la base de la dispersion des valeurs arrondies d'AMPC calculés alentour de chacune des valeurs d'AMPC affectés (Figure 7, bas), j'ai donc pu estimer les proportions (%) de chacune des éventuelles souches (azoto)bactériennes, avantageusement endogènes, provenant des divers AMPC affectés constituant un consortia le mieux adapté à un AMPC affecté en particulier. A noter que pour que cette dispersion (imprécision) serve à borner les gammes d'AMPC affectés, ici sans application des if, sources de souches (azoto)bactérienne, il est avantageux que les valeurs AMPC calculés mais non arrondies soient distribuées plutôt normalement alentour de chacune des AMPC affectés correspondants. Cette condition est ici la mieux respectée pour les AMPC 4, 5, 6, 7 et 8, soit les plus usuelles étant données les classes argileuses aux quelles elles appartiennent.

Étant donnée l'imperfection des proportions rapportées au Tableau 4, il est possible de raffiner cette approche en remplaçant les AMPC affectés par des valeurs pondérées localement à l'aide d'un indice dit IDW (inverse distance weighting ; Lloyd 2011, p156). Cette technique implique que par rapport à la valeur au point central i = 0, il est donc possible de calculer précisément la distance de chacun des 12 point par rapport à celui-ci. Cette distance, dite $d_{i0}$, servira à calculer une autre valeur centrale pondérée selon la formule suivante ;

$$\text{valeur pondérée si } i = 0 \ (\check{z}) = [(\Sigma \ z(Si) \ d_{i0}^{-r}) \ / \ \Sigma \ d_{i0}^{-r}] \qquad (2)$$

➢ z(Si) est la valeur du paramètre, par exemple NNI, MOBN ou MSPA pour chacun de i points d'échantillonnage

➢ $d_{i0}$ est la distance de ce points d'échantillonnage par rapport à i = 0

➢ r est un facteur de pondération (weighting) explicitant l'effet de l'éloignement, $d_{i0}$, des i échantillons par rapport à i = 0.

➢ n = nombre d'observations (moyennes cantonales), soit ici 5 par cadre bande d'application.

Il est en principe plus réaliste de remplacer le terme $d_{i0}^{-r}$ par une variable plus *gaussienne* (Wij ; Lloyd 2011, p74) tel que ;

$$W_{ij} = \exp[-0.5\,[d/T]^2] \qquad\qquad (3)$$

> d est la distance euclidienne entre l'observation i et la position voisine j,

> T est la "largueur" de la bande d'application, soit 1/5 de la distance géographique applicable à la distance génomique des populations bactériennes du sol (cf. Cho et Tiedje 2000), soit approximativement ici 1/5 du diamètre d'un département français moyen,

De cette manière, la valeur d'AMPC désigné deviendra une valeur plus régulière et progressive comparable en sorte à celles des AMPC calculés (Figure 8) ; l'étendue des gamme d'AMPC calculés comprise par chacune de ces AMPC pondérée est aussi dans ce cas plus petite, et les consortium à concevoir pour les parcelles agronomiques correspondantes d'autant plus facile à réaliser. Dans ce cas ci, la régression linéaire multivariée obtenue est la suivante ;

$$AMPC_{calculé} = 0{,}0559[\text{argile}] - 0{,}0913[C_{organique}] - 0{,}3203[pH_{eau}] - 0{,}1651\ ;\ R^2\ 93\% \qquad (4)$$

Il faut cependant ici arrondir ces valeurs d'AMPC affectés continues (Figure 9) en valeurs elles aussi discrètes de manière à retrouver un schème applicable comme celui à la Figure 7. Ces nouvelles proportions (Tableau 5) tiennent maintenant compte de la pondération géostatistique selon l'équation (2) ci-dessus.

**Tableau** 5 : Composition des consortia bactériens endogène adaptées aux AMPC affectés (v2).

| AMPC | | Calculés ➔ % des souches membre du consortium adapté à l'AMPC affecté selon leur origine | | | | | | | | | | | |
|------|---|-----|-----|-----|-----|-----|-----|-----|-----|-----|-----|-----|-----|
| | | 1 | 2 | 3 | 4 | 5 | 6 | 7 | 8 | 9 | 10 | 11 | 12 |
| Affectés | 1 | 7% | 13% | 30% | 50% | | | | | | | | |
| | 2 | 7% | 17% | 45% | 31% | | | | | | | | |
| | 3 | 1% | 5% | 18% | 49% | 27% | | | | | | | |
| | 4 | | 2% | 4% | 23% | 60% | 10% | | | | | | |
| | 5 | | | 1% | 8% | 63% | 28% | 1% | | | | | |
| | 6 | | | | | 6% | 68% | 24% | 3% | | | | |
| | 7 | | | | | 2% | 33% | 43% | 16% | 5% | | | |
| | 8 | | | | | | 9% | 42% | 38% | 8% | 3% | | |
| | 9 | | | | | | | 2% | 58% | 33% | 5% | 2% | |
| | 10 | | | | | | | | 6% | 44% | 35% | 12% | 3% |
| | 11 | | | | | | | | | 8% | 41% | 34% | 17% |
| | 12 | | | | | | | | | | 4% | 33% | 64% |

Comme on peut le voir, ces proportions au Tableau 5 sont maintenant moins incongrues et plus facilement réalisables que celles au Tableau 4. En effet, l'équation (4) est plus précise que celle obtenue sans pondération (cf. équation 1).

Enfin, a noter aussi qu'en pratique il n'est pas absolument nécessaire d'avoir autant de consortia que d'AMPC. Par exemple, en respectant les proportions aux le Tableau 5, un *seul* consortium de quatre (4) souches provenant de quatre d'AMPC affectés *différents* selon les seuils minimax aux Tableau 2 ou 3 sera applicable sur *plusieurs* AMPC affectés. Par exemple, la réalisation de l'invention pourrait ce faire simplement en concevant que quatre (4) consortia (azoto)bactériens, à savoir ;

consortia 1 : souches provenant des $AMPC_{affectés}$ 2 à 5    → parcelles $AMPC_{affectés}$ 1 à 3

consortia 2 : souches provenant des $AMPC_{affectés}$ 4 à 7    → parcelles $AMPC_{affectés}$ 4 à 6

consortia 3 : souches provenant des $AMPC_{affectés}$ 6 à 9    → parcelles $AMPC_{désigné}$ 7 à 9

consortia 4 : souches provenant des $AMPC_{affectés}$ 9 à 12    → parcelles $AMPC_{affectés}$ 10 à 12

pour couvrir en sorte l'ensemble de la SAU ainsi segmentée en quatre classes argileuses comprenant 12 AMPC. C'est l'imprécision inhérente à cette régression qui permet ainsi de former et proportionner les consortia les mieux adaptés à une AMPC affecté en particulier.

***Références***

Afes (Association française pour l'étude du sol). 2009. Référentiel pédologique. Eds. Quae, 2009, RD10, 78026 Versailles, Fance

Carson, JK, V Gonzalez-Quinones, DV Murphy, C Hinz, JA. Shaw et DB Gleeson. 2010. Low Pore Connectivity Increases Bacterial Diversity in Soil. Appl. Environ. Microbiol. 76 (12) : 3936-3942

Cho, J-C et JM Tiedje. 2000. Biogeography and Degree of Endemicity of Fluorescent Pseudomonas Strains in Soil. Appl. Environ. Microbiol., Dec. 2000, p. 5448-5456 Vol. 66, No. 12

Claude, PP et L Fillon. 2004. Effet de l'apport d'un inoculum bactérien aux résidus de culture de maïs-grain au sol sur le rendement et la qualité de blés d'hiver panifiables en France. Agrosol. (depuis - Agrosolutions) 15 (1) : 23-29

Coleman, K et DS Jenkinson. 2008. RothC-26.3, A model for the turnover of carbon in soil : models description and Windows users guide. Rothamsted Research, Harpenden, Herts, UK, AL5 2JQ.

Curtis, TP, WT Sloan et JW Scannell. 2002. Estimating prokaryotic diversity and its limits. PNAS, pp. 10494-10499, August 6, 2002, vol. 99, no. 16

Dumanski, J., W.W. Pettapiece, D.F. Acton et P.P. Claude. 1993. Application of agro-ecolie/ce concepts and hierarchy theory in the design of databases for spatial and temporal characterisation of land and soil. Geoderma, 60 (1993) 343-358 343

Fierer, N. MA Bradford et RB Jackson. 2007. Toward an ecological classification of soil bacteria. Ecology, 88(6), 2007, pp. 1354-1364

Fierer, N., MA. Bradford, et RB. Jackson. 2007. Toward An Ecological Classification Of Soil Bacteria. Ecology, 88(6), 2007, pp. 1354-1364

Fox, GA et R Metla. 2005. Soil Property Analysis using Principal Components Analysis, Soil Line, and Regression Models. Soil Sci. Soc. Am. J. 69:1782-1788.

Fox, GA et GJ Sabbagh. 2002. Estimation of Soil Organic Matter from Red and Near-Infrared Remotely Sensed Data Using a Soil Line Euclidean Distance Technique. Soil Sci. Soc. Am. J. 66:1922-29.

Franklin, RB et AL Mils. 2003. Multi-scale variation in spatial heterogeneity for microbial community structure in an eastern Virginia agricultural field. FEMS Microbiology Ecology 44 : 335-346

Gaillard et al. 1999. Carbon, nitrogen and microbial gradients induced by plant residues decomposing in soil. Eur. J. Soil Sci. 50 : 567-578

Gaillard et al. 2003. Carbon minéralisation in soil adjacaent to plant residues of contrasting biochemical quality. Soil Biol. Biochem. 35 : 93-99

Garey A. Fox and Roopa Metla. 2005. Soil Property Analysis using Principal Components Analysis, Soil Line, and Regression Models. Soil Sci. Soc. Am. J. 69:1782-1788 (2005).

GISSOL (Groupement d'intérêt scientifique sol). 2006. Base de Données Analyse des Terres. 1 mars 2006, XP002699913, URL: http://www.gissol.fr/programme/bdat/bdat. php

GrunAMPCann, G. L. et P. Normand. 2000. Microscale Diversity of the Genus Nitrobacter in Soil on the Basis of Analysis of Genes Encoding rRNA. Appl. Environ. Microbiol., Oct. 2000, p. 4543-4546 Vol. 66, No. 10

Halsall, DM et AH Gibson 1991. Nitrogenase activity in straw-amended wheat belt soils in response to diazotrophe inoculation. Soil Biology and Biochemistry 23(10) : 987-998.

Husain et al. 2011. Kinetic Study of a Bacterial Consortium Isolated from Soil Contaminated with Crude Oil. Australian Journal of Basic and Applied Sciences, 5(6): 925-930

Jacques et al. 2008. Utilisation de consortia fongi - bactériens pour la dégradation in situ de composés poly-aromatiques (PAH). Biores. Technol. 99(7) : 2637-43

Jamagne, M. 2011. Grands paysages pédologiques de la France. Éditions Quae, RD 10, 78026 Versailles Cedex (France).

Kadono, A. et al. 2009. Factors controlling potentially mineralizable and récalcitrant soil organic matter in humid Asia. Soil. Sci. Plant. Nutr. 55 (2) : 243-251

Lebart, L, A Morineau et M. Piron. 2000. Statistique exploratoire multidimensionnelle. 3ième Édition. Dunod, Paris

Lloyd, CD. 2011. Local models for spatial analysis (2nd Edition). CRC Press, New York.

Mendes et al. 2011. Dechipering ring the Rhizosphere Microbiome for Disease-Suppressive Bacteria. Science 332 : 1097

Martiny, JBH, JA Eisen, K Penn, SD Allison et M-C Horner-Devine. 2011. Drivers of bacterial $\beta$-diversity depend on spatial scale. PNAS, pp. 7850-7854 May 10, 2011 | vol. 108 | no. 19

McBratney, Alex. B. Inakwu et O.A. Odeh. 1997. Application of fuzzy sets in soil science: fuzzy logic, fuzzy measurements and fuzzy decisions. Geoderma 77 (1997) 85-113

Moreno, J, T. De La Rubia, A Ramos-Ormenzana et GR Vela. 1990. Growth and nitrogenase activity of Azotobacter vinelandii on soil phenolic acids. Journal of Applied Bacteriology 69 : 328.

Quinn, JA, DB McKay et B Entsch. 2001. Analysis of the pobA and pobR genes controlling expression of p-hydroxybenzoate hydroxylase in Azotobacter chroococcum, Gene 264 : 77-85.

Radianingtyas et al. 2003. Characterization of a soil-derived bacterialconsortium degrading 4-chloroaniline. Microbiology 149 : 3279-3287

Ramette, Alban et James M. Tiedje. 2007. Multiscale responses of microbial life to spatial distance and environmental heterogeneity in a patchy ecosystem. PNAS 104 : 8 2761-2766

Richaume et al. 2006. Évaluation des modifications quantitatives, qualitatives et fonctionnelles induites par la conservation de consortiums bactériens extraits de sols. Les Actes du BRG, 6 (2006) 371-389

Roper MM, RR Gault et NA Smith. 1995. Contribution to the N status of soil by free living nitrogen fixing bacteria in a lucerne stand. Soil Biol. Biochem. 27(4/5) : 467-471

Sessitsch, A, A Weilharter, MH. Gerzabek, H Kirchmann et E Kandeler. 2001. Microbial Population Structures in Soil Particle Size Fractions of a Long-Term Fertilizer Field Experiment. Appl. Environ. Microbiol. 67(9) : 4215-4224

Tran, L.T. et al. 2002. Application of fuzzy logic-based modeling to improve the performance of the Revised Universal Soil Loss Equation. CATENA 47 (3) : 203-226

Tran, L.T., M.A. Ridgley, M.A. Nearing, L. Duckstein et R. Sutherland. 2001. Using fuzzy logic based modelling to improve the performance of the revised universal sol loss equation. In : "Sustaining the Global Farm". D.E. Stott, R.H. Mohtar et G.C. Steinhardt (eds). USDA-ARS / Purdue University.

Turner, BL, BJ Cade-Menun et DT Westermann. 2003. Organic Phosphorus Composition and Potential Bioavailability in Semi-Arid Arable Soils of the Western United States. Soil Sci. Soc. Am. J. 67:1168-1179

Van der Gucht, K, K Cottenie, K Muylaert Vloemans, N, S Cousin, S Declerck, E Jeppesen, J-M Conde-Porcuna, K Schwenk, G Zwart, H Degans, W Vyverman et L De Meester. 2007. The power of species sorting: Local factors drive bacterial community composition over a wide range of spatial scales. PNAS 104 (51) : 20404-20409

Vogel, J., P. Normand, J. Thioulouse, X. Nesme et G. L. GrunAMPCann. 2003. Relationship between Spatial and Genetic Distance in Agrobacterium spp. in 1 Cubic Centimeter of Soil. Appl. Environ. Microbiol., Mar. 2003, p. 1482-1487 Vol. 69, No. 3

Zadeh, L.A. 1965. Fuzzy sets. Information and Control 8 : 338-353.

Zhu, X., B. Hudson, J. Burt, K. Lubich et D. Simonson. 2001. Soil Mapping Using GIS, Expert Knowledge, and Fuzzy Logic. Soil Sci. Soc. Am. J. 65:1463-1472.

**Revendications**

1. **Procédé d'affectation d'un *agrolmicro* - *pédoclimat* (AMPC) à une parcelle et/ou un îlot agricole et formation de consortia (azoto)bactériens biofertilisants** par transcription de variables pédologiques descriptives et d'un échantillon de terre arable en variables agro/micro-pédologiques comprenant les étapes suivantes:

   a) l'analyse physico-chimique d'un ensemble d'échantillons de terre de référence afin d'effectuer une classification de ces échantillons sur la base de leurs teneurs en argile, créant ainsi une gamme de n classes argileuses ;
   b) au sein d'une même classe argileuse, l'analyse physico-chimique préalable d'un ensemble d'échantillons de terre afin d'extraire de cet ensemble une série de composantes principales, alias CPn, pouvant être mises en relation avec l'une ou l'autre des susdites variables pédologiques ;
   c) la segmentation desdites classes argileuses en un nombre q d'agro/micro - pédoclimat, alias AMPC, à l'aide de seuils minimax obtenus par mise en relation desdites variables pédologiques et des deux premières CPn, CP1 et CP2, les plus corrélées sachant que,

   ➢ les CPn sont obtenues par *analyse* multivariée, y compris ici avantageusement par *composantes principale* (ACP),

   ➢ les desdites CPn sont mises en relation par régressions linéaires [$VAR_{1/2}$ : $CP_{1/2}$], où $VAR_{1/2}$ sont les deux variables pédologiques les plus corrélées aux deux premières *composantes principales,* $CP_{1/2}$, respectivement, issues de ladite analyse multivariée,

   ➢ les n classes argileuses sont segmentées en q AMPC à l'aide de *seuils minimax* applicables à chacune des classes argileuses, cette segmentation donnant un nombre n x q d'AMPC attribuables, et enfin que,

   ➢ sachant que les seuils minimax $MiniMax_{1/2}$ applicables à chacune des *n* - classes d'observations sont déterminés par la formule suivante -

$$[\text{MinMax}_{1/2} = \text{moyenne de VAR}_{1/2} +/- (\text{pente de la régression linéaire } [\text{VAR}_{1/2} : \text{CP}_{1/2}] \text{ x } R^2_{1/2})]$$

lorsque $VAR_{1/2}$ sont les deux variables pédologiques, hormis celles ayant servi à la classification en groupes homogènes suite à l'ACP, les plus corrélées avec $CP_{1/2}$, *i.e.* les coordonnées des deux premières composantes principales des $n$ - ACP, respectivement, et $R^2_{1/2}$ les deux coefficients de déterminations desdites régression linéaires [$VAR_{1/2}$ : $CP_{1/2}$], respectivement ;

d) l'échantillonage de la parcelle et/ou l'îlot agricole pour fin d'analyse physico-chimique ;

e) l'analyse physico-chimique de l'échantillon de terre de la parcelle et/ou îlot agricole générant des variables pédologiques afin de lui associer une description physico-chimique comprenant au moins les variables, teneur en argile et en carbone organique, pH eau ou KCl, voire la CEC et/ou la granulométrie ;

f) l'attribution d'une classe argileuse à cet échantillon de terre, et donc par inférence à ladite parcelle et/ou îlot agricoles desquels il provient ;

g) l'affectation d'un AMPC à l'échantillon de terre par, (i) comparaison de sa teneur en argile afin de le situer dans une classe argileuse particulière, et (ii) par comparaison de son pH ou sa teneur en carbone organique afin de déterminer leur position par rapports aux susdits seuils minimax ;

la formation des consortia (azoto)bactériens biofertilisants comportant le calcul des valeurs $AMPC_{calculé}$ à l'aide d'une régression linéaire multivariée des teneurs en argile et $C_{organique}$ et le $pH_{eau}$ comme variables explicatives, selon la formule suivante :

eau

$$AMPC_{calculé} = a \cdot [\text{argile}] - b \cdot [C_{organique}] + c \cdot [pH_{eau}] - d$$

et la comparaison des AMPFC affectés et calculés, et

h) l'analyse d'un ensemble d'échantillons de terre de référence obtenus à l'aide des susdites analyses physicochimiques et établissement ainsi d'une relation multivariée empirique entre les susdites valeurs d'AMPC affectés et les valeurs des variables pédologiques les plus impliquées dans la détermination des niveaux d'activité et/ou de diversité des populations azotobactériennes du sol ;

i) la comparaison graphique et/ou mathématique de ces AMPC calculés et affectés afin d'obtenir une appréciation de la dispersion des AMPC calculés alentour des AMPC affectés sachant que,

◦ les valeurs des AMPC ainsi calculés pour chacune des valeurs AMPC affectés sont distribuées normalement,

◦ les valeurs des AMPC ainsi calculés sont arrondies à des nombres entiers,

◦ la fréquence et/ou la densité de probabilité associée à chacune des valeurs des AMPC calculés ainsi arrondies détermine la proportion relative dans le consortia des souches (azoto)bactériennes provenant des AMPC affectés ;

j) la formation, à titre de consortia, pour chacun des AMPC affectés des gammes de souches (azoto)bactériens provenant d'AMPC affectés compris dans cette dispersion d'AMPC calculés alentour de chacun de ces valeurs d'AMPC affectés.

2. **Procédé d'affectation d'un *agro/micro* - *pédoclimat* (AMPC) à une parcelle et/ou un îlot agricole et de formation de consortia (azoto)bactérien biofertilisants** selon la première revendication précédente **caractérisé en ce que** l'application des seuils minimax est plutôt conditionnée par la *co-inertie* entre les variables VAR1 et 2 permettant d'établir des *intervalles floues* (if) déterminées comme suit ;

$$\text{if} = \left[(\text{coordonnée CP1} = R^2 \mid [\text{VAR}_1 : \text{CP}_1]) : (\text{coordonnée CP2} = R^2 \mid [\text{VAR}_2 : \text{CP}_2])\right]$$

ces intervalles de confiances étant ainsi applicables directement aux régressions [$VAR_{1/2}$ : $CP_{1/2}$] de manière à identifier la plage de valeurs que peuvent prendre VAR1 et 2 observées pour les susdits échantillons de terre arable.

3. **Procédé d'affectation d'un *agro/micro* - *pédoclimat* (AMPC) à une parcelle et/ou un îlot agricole et de for-**

**mation de consortia (azoto)bactérien biofertilisants** selon l'une quelconque des revendications précédentes **caractérisé en ce que** la formation des classes argileuses se fait à l'aide d'une classification ascendante hiérarchique d'un ensemble préalable d'échantillons de terre.

4. **Procédé d'affectation d'un** *agro/micro - pédoclimat* **(AMPC) à une parcelle et/ou un îlot agricole et de formation de consortia (azoto)bactérien biofertilisants** selon l'une quelconque des revendications précédentes **caractérisé en ce que** le nombre de classes argileuses est compris entre 2 et 20, plus particulièrement entre 3 et 16, et avantageusement ici de 4.

5. **Procédé d'affectation d'un** *agro/micro - pédoclimat* **(AMPC) à une parcelle et/ou un îlot agricole et de formation de consortia (azoto)bactérien biofertilisants** selon l'une quelconque des revendications précédentes **caractérisé en ce qu'**au moins deux échantillons de terre géographiquement distants d'environs 80 kms ou plus se voient attribuer un même AMPC.

6. **Procédé d'affectation d'un** *agro/micro - pédoclimat* **(AMPC) à une parcelle et/ou un îlot agricole et de formation de consortia (azoto)bactérien biofertilisants** selon l'une quelconque quelconque des revendications précédentes **caractérisé en ce que** la relation multivariée empirique est une régression linéaire ou non - linéaire.

7. **Procédé d'affectation d'un** *agro/micro - pédoclimat* **(AMPC) à une parcelle et/ou un îlot agricole et de formation de consortia (azoto)bactérien biofertilisants** selon l'une quelconque des revendications précédentes **caractérisé en ce que** les variables pédologiques les plus impliquées dans la détermination du niveau d'activité et/ou de diversité des populations azotobactériennes du sol sont choisies parmi un groupe comprenant le pH, les teneurs en argile et en carbone organique, voire le CEC et/ou la granulométrie.

**Patentansprüche**

1. **Methode zur Zuordnung eines agrar/mikro-Bodenklimas (AMBK) zu einer Parzelle und/oder einer landwirtschaftlichen Fläche und zur Bildung von biofertilisierenden (Azoto-) Bakterienkonsortien** durch die Transkription deskriptiver bodenkundlicher Variablen einer Ackerbodenprobe in agrar/mikro-bodenkundliche Variablen, einschließlich folgender Schritte:

   a) Die physikalisch-chemische Analyse einer Reihe von Referenz-Bodenproben, um eine Klassifikation dieser Proben anhand ihres Tongehalts durchzuführen, um so eine Reihe von n Tonklassen zu erstellen;
   b) innerhalb derselben Tonklasse kann die vorherige physikalisch-chemische Analyse eines Satzes von Bodenproben, aus der eine Reihe von Hauptkomponenten, auch als CPn bezeichnet, abgeleitet wird, mit einer der oben genannten Bodenvariablen verknüpft werden;
   c) die Unterteilung dieser Tonklassen in eine Anzahl q von agrar/mikro-Bodenklimata, alias AMBK, mit der Hilfe von Minimax-Schwellenwerten, die man aus der Verknüpfung dieser bodenkundlichen Variablen und der beiden ersten CPn, CP1 und CP2, den am besten korrelierenden, erhält, wobei zu berücksichtigen ist, dass

   ➢ die CPn durch multivariate *Analyse* erhalten werden und hier vorteilhafterweise einschließlich der *Hauptkomponenten* (ACP),

   ➢ die vorgenannten CPn durch lineare Regression verknüpft werden, wobei [$VAR_{1/2}$ : $CP_{1/2}$], oder $VAR_{1/2}$ die beiden bodenkundlichen Variablen sind, die am stärksten mit den ersten beiden *Hauptkomponenten,* $CP_{1/2}$, respektive, die sich aus der besagten multivariaten Analyse ergeben haben, korrelieren,

   ➢ die n Tonklassen mit der Hilfe von Minimax-Schwellenwerten, die für jede der Tonklassen zutreffen, in q AMBK unterteilt werden; diese Unterteilung ergibt eine Anzahl von n x q anwendbaren AMBK, und schließlich, dass

   ➢ im Wissen, dass die MiniMax-Schwellenwerte $MiniMax_{1/2}$, die für jede der n Beobachtungsklassen gelten, durch die folgende Formel bestimmt sind -

   $$[MinMax_{1/2} = \text{Mittelwert von } VAR_{1/2} +/- (\text{Steigung der linearen Regression } [VAR_{1/2} : CP_{1/2}] \times R^2_{1/2})]$$

   wenn $VAR_{1/2}$ die beiden bodenkundlichen Variablen sind, mit der Ausnahme jener, die zur Klassifikation in homogene Gruppen folgend der ACP verwendet wurden, die am stärksten mit $CP_{1/2}$ korrelieren, *i.e.* die Koordinaten der beiden ersten Hauptkomponenten der $n$ - ACP, respektive, und $R^2_{1/2}$ die beiden Bestimmungs-Koeffizienten der linearen Regression $[VAR_{1/2}: CP_{1/2}]$, respektive;

d) die Probenentnahme aus der Parzelle und/oder landwirtschaftlichen Fläche für die physikalisch-chemische Analyse;

e) die physikalisch-chemische Analyse der Bodenprobe der Parzelle und/oder landwirtschaftlichen Fläche ergibt bodenkundliche Variablen, um eine physikochemische Beschreibung zuzuordnen, die zumindest die Variablen Gehalt an Ton und organischem Kohlenstoff, Wasser-pH oder KCl enthält oder sogar die KAK und/oder die Korngröße;

f) die Zuordnung dieser Bodenprobe zu einer Tonklasse, und damit auch durch Rückschluss auf die Parzelle und/oder der landwirtschaftlichen Fläche, von der sie stammt;

g) die Zuordnung eines AMBK zu dieser Bodenprobe für (i) den Vergleich ihres Tongehalts, um sie in eine bestimmte Tonklasse einordnen zu können, und (ii) für den Vergleich ihres pH-Wertes oder ihres Gehalts an organischem Kohlenstoff, um ihre Position in Bezug auf die oben genannten Mindestgrenzen zu bestimmen; die Bildung von biofertilisierenden (Azoto-) Bakterienkonsortien, die die Berechnung der $AMBK_{berechnet}$-Werte, unter Verwendung einer multivariaten linearen Regression der Gehalte an Ton und $C_{organisch}$ sowie des pHwasser als erklärende Variablen, umfasst, nach der folgenden Formel:

$$AMBK_{berechnet} = a \cdot [Ton] - b \cdot [C_{organisch}] + c \cdot [pH_{Wasser}] - d$$

sowie der Vergleich der zugeordneten und berechneten AMBK, und

h) die Analyse einer Reihe von Referenz-Bodenproben, die mittels der erwähnten physikalisch-chemischen Analysen und der darauffolgenden Herstellung einer empirischen multivariaten Beziehung zwischen den obigen Werten des zugeordneten AMBK und den Werten der bodenkundlichen Variablen, die am wichtigsten sind für die Bestimmung der Aktivitätsniveaus und/oder der Diversität der azotobakteriellen Populationen im Boden;

i) der grafische und/oder mathematische Vergleich der berechneten und des zugeordneten AMBK, um eine Einschätzung der Streuung der berechneten AMBK um die zugeordneten AMBK zu erhalten, in dem Wissen, dass

○ die so berechneten AMBK-Werte für jeden der zugeordneten AMBK-Werte normalverteilt sind,
○ die so berechneten AMBK-Werte auf ganze Zahlen gerundet sind,
○ die Häufigkeit und/oder die Wahrscheinlichkeitsdichte, die mit jedem dieser so gerundeten berechneten AMBK-Werte verbunden ist, den relativen Anteil in den Konsortien der echten (Azoto-)Bakterien bestimmt, die vom zugeordneten AMBK stammt;

j) die Bildung, als Konsortien, für jede der zugeordneten AMBK von Gruppen von echten (Azoto-) Bakterien, stammen von den zugeordneten AMBK, die in dieser Streuung des berechneten AMBK um jeden der Werte des zugeordneten AMBK enthalten sind.

2. **Verfahren zur Zuordnung eines agrar/mikro-Bodenklimas (AMBK) zu einer Parzelle und/oder einer landwirtschaftlichen Fläche und zur Bildung von biofertilisierenden (Azoto-) Bakterienkonsortien** gemäß dem ersten, vorhergehenden Anspruch, der **dadurch gekennzeichnet ist, dass** die Anwendung von MiniMax-Schwellenwerten eher durch die Ko-Trägheit zwischen den Variablen VAR1 und 2 bestimmt wird, wodurch ermöglicht wird, fuzzy Intervalle (fi) einzuführen, die wie folgt definiert sind;

$$fi = \big[(Koordinate\ CP1 = R^2 \mid [VAR_1 : CP_1]) : (Koordinate\ CP2 = R^2 \mid [VAR_2 : CP_2])\big]$$

diese Konfidenzintervalle sind so auch direkt auf die Regressionen anzuwenden $[VAR_{1/2} : CP_{1/2}]$ um den Wertebereich zu identifizieren, den VAR1 und 2 für die beschriebenen Agrarboden-Proben annehmen können.

3. **Verfahren zur Zuordnung eines agrar/mikro-Bodenklimas (AMBK) zu einer Parzelle und/oder einer landwirtschaftlichen Fläche und zur Bildung von biofertilisierenden (Azoto-) Bakterienkonsortien** gemäß einem der vorhergehenden Ansprüche, der **dadurch gekennzeichnet ist, dass** die Bildung der Tonklassen mithilfe einer aufsteigenden hierarchischen Klassifikation durchgeführt wird, die aus einem vorherigen Satz von Bodenproben stammt.

**4.** **Verfahren zur Zuordnung eines agrar/mikro-Bodenklimas (AMBK) zu einer Parzelle und/oder einer landwirtschaftlichen Fläche und zur Bildung von biofertilisierenden (Azoto-) Bakterienkonsortien** gemäß einem der vorhergehenden Ansprüche, der **dadurch gekennzeichnet ist, dass** die Anzahl der Tonklassen zwischen 2 und 20 liegt, genauer zwischen 3 und 16 und am vorteilhaftesten hier bei 4.

**5.** **Verfahren zur Zuordnung eines agrar/mikro-Bodenklimas (AMBK) zu einer Parzelle und/oder einer landwirtschaftlichen Fläche und zur Bildung von biofertilisierenden (Azoto-) Bakterienkonsortien** gemäß einem der vorhergehenden Ansprüche, der **dadurch gekennzeichnet ist, dass** zumindest zwei geografisch weit entfernten Bodenproben, von 80 km oder mehr Entfernung, das gleiche AMBK zugewiesen wird.

**6.** **Verfahren zur Zuordnung eines agrar/mikro-Bodenklimas (AMBK) zu einer Parzelle und/oder einer landwirtschaftlichen Fläche und zur Bildung von biofertilisierenden (Azoto-) Bakterienkonsortien** gemäß einem der vorhergehenden Ansprüche, der **dadurch gekennzeichnet ist, dass** die empirische multivariate Beziehung eine lineare oder nicht-lineare Regression ist.

**7.** **Verfahren zur Zuordnung eines agrar/mikro-Bodenklimas (AMBK) zu einer Parzelle und/oder einer landwirtschaftlichen Fläche und zur Bildung von biofertilisierenden (Azoto-) Bakterienkonsortien** gemäß einem der vorhergehenden Ansprüche, der **dadurch gekennzeichnet ist, dass** die wesentlichsten bodenkundlichen Variablen für die Bestimmung des Aktivitätsniveaus und/oder der Diversität der azotobakteriellen Populationen im Boden ausgewählt werden aus einer Gruppe, die den pH-Wert, den Gehalt an Ton und an organischem Kohlenstoff, oder sogar den KAK und/oder die Korngröße enthält.

**Claims**

**1.** **Method of assigning an agro/micro-pedoclimate (AMPC) to an agricultural plot and/or islet and the formation of biofertilizing (azoto)bacterial consortia** by transcription of descriptive variables for a sample of arable land into agro/micro-pedoclimatic variables comprised of the following steps;

a) the physico-chemical analysis of a set of reference soil samples in order to classify these samples on the basis of their clay contents, thus creating a range of $n$ clay classes;

b) within the same clay class, the prior physico-chemical analysis of a set of soil samples in order to extract from this set a series of principal components, alias CPn, which can be related to either of the above soil variables;

c) the segmentation of said clay classes into a number q of agro/micro-pedoclimates, alias AMPC, using minimax thresholds obtained by relating the aforesaid soil variables and the first two CPn most correlated, CP1 and CP2, knowing that,

• the CPn are obtained by multivariate analysis, including here advantageously by principal components analysis (PCA),

• the said CPn are correlated by linear regressions [$VAR_{1/2}$: $CP_{1/2}$], where $VAR_{1/2}$ are the two soil variables most correlated with the first two main components, $CP_{1/2}$, respectively, resulting from said multivariate analysis,

• the $n$ clay classes are segmented into q AMPCs using minimax thresholds applicable to each of the clay classes, this segmentation giving an $n$ x $q$ number of attributable AMPCs, and finally that,

• knowing that the $MiniMax_{1/2}$ minimax thresholds applicable to each of the n - observation classes are determined by the following formula -

$$[MinMax_{1/2} = \text{mean of } VAR_{1/2} +/- (\text{slope of the linear regression } [VAR_{1/2}: CP_{1/2}] \times R^2_{1/2})]$$

where $VAR_{1/2}$ are the two soil variables, apart from those used for classification into homogeneous groups by PCA, the most correlated with $CP_{1/2}$, i.e. the coordinates of the first two main components of the $n$ - PCAs, respectively, and $R^2_{1/2}$ the two coefficients of determination of the said linear regression [$VAR_{1/2}$: $CP_{1/2}$], respectively;

d) sampling of the plot and / or the agricultural block for the purpose of physico-chemical analysis;

e) physico-chemical analysis of the soil sample of the agricultural plot and/or islet generating soil variables in order to associate a physico-chemical description comprised of at least the variables, clay and organic carbon content, pH in water or KCl, or again CEC and/or particle size distribution (granulometry);

f) the attribution of a clay class to this sample of soil, and therefore by inference to said plot and / or agricultural block from which it comes;

g) assigning an AMPC to the soil sample by, (i) comparing its clay content in order to place it in a particular clay class, and (ii) by comparing its pH or its organic carbon content in order to determine their position in relation to the above minimax thresholds;

the formation of biofertilizing bacterial consortia (azoto) including the calculation of AMPC values calculated using a multivariate linear regression of clay and Corganic contents and pH water as explanatory variables, according to the following formula;

$$\text{AMPC}_{calculated} = a \cdot [clay] - b \cdot [Corganic] + c \cdot [pHeau] - d$$

the comparison of assigned and calculated AMPCs, and

h) the analysis of a set of reference soil samples obtained using the above physicochemical analyses and the establishment of an empirical multivariate relationship between the above assigned AMPC values and the values of the most soil variables involved in determining the activity and / or diversity levels of soil azotobacterial populations;

i) the graphic and / or mathematical comparison of these calculated and assigned AMPC in order to obtain an appreciation of the dispersion of the calculated AMPC around the affected AMPC, knowing that,

- the AMPC values thus calculated for each of the assigned AMPC values are distributed normally,
- the AMPC values thus calculated are rounded to whole numbers,
- the frequency and / or the probability density associated with each of the values of the calculated AMPC thus rounded determines the relative proportion in the consortia of (azoto)bacterial strains originating from the assigned AMPC;

j) for each of the assigned AMPC, the formation of consortia of (azoto)bacterial strains originating from assigned AMPC contained within this dispersion of calculated AMPC around each of these assigned AMPC values.

2. **Method of assigning an agro/micro-pedoclimate (AMPC) to an agricultural plot and/or islet and the formation of biofertilizing (azoto)bacterial consortia** according to the first and preceding claim **characterized in that** the application of the minimax thresholds is instead conditioned by the co-inertia between the variables VAR1 and 2 making it possible to establish fuzzy intervals (if) determined as follows;

$$\text{if} = [(\text{coordinate CP1} = R^2 \mid [\text{VAR1: CP1}]): (\text{coordinate CP2} = R^2 \mid [\text{VAR2: CP2}])]$$

these confidence intervals thus being directly applicable to the regressions [VAR1/2: CP1/2] so as to identify the range of values that the observed VAR1 and 2 can take for the aforementioned samples of topsoil.

3. **Method of assigning an agro/micro-pedoclimate (AMPC) to an agricultural plot and/or islet and the formation of biofertilizing (azoto)bacterial consortia** according to any one of the preceding claims, **characterized in that** the formation clay classes is done using an ascending hierarchical classification of an existing set of soil samples.

4. **Method of assigning an agro/micro-pedoclimate (AMPC) to an agricultural plot and/or islet and the formation of biofertilizing (azoto)bacterial consortia** according to any one of the preceding claims, **characterized in that** the number of clay classes is between 2 and 20, more particularly between 3 and 16, and, here, advantageously 4.

5. **Method of assigning an agro/micro-pedoclimate (AMPC) to an agricultural plot and/or islet and the formation of biofertilizing (azoto)bacterial consortia** according to any one of the preceding claims, **characterized in that** at least two geographically distant soil samples geographically distant by approximately 80 km or more are assigned the same AMPC.

6. **Method of assigning an agro/micro-pedoclimate (AMPC) to an agricultural plot and/or islet and the formation of biofertilizing (azoto)bacterial consortia** according to any one of the preceding claims, **characterized in that** the empirical multivariate relationship is a linear or nonlinear regression.

7. **Method of assigning an agro/micro-pedoclimate (AMPC) to an agricultural plot and/or islet and the formation of biofertilizing (azoto)bacterial consortia** according to any one of the preceding claims, **characterized in that** the soil variables determining the most the level of activity and/or the diversity of soil azotobacterial populations are chosen from a group comprising pH, clay and organic carbon contents, or again CEC and/or particle size distribution (granulometry).

Figure 1

**Figure 2**

**eg. classe argileuse 1 / pH selon CP1**

AMPC II   AMPC I   AMPC III

CP1

Figure 3

%Argile ?

ca1   ca2   ca3   ...   $ca_n$

Variable 1 (VAR1) selon CP-1

Minima   Maxima

Variable 2 (VAR2) selon CP-2

Minima   Maxima

AMPC/3-II   AMPC/3-I   AMPC/3-III

Figure 4

Variables pédologiques selon CP 1 et 2

Composantes principales ACP 1 et 2

## Figure 5

$$y = -0,220x + 6,332$$
$$R^2 = 0,530$$

composantes principale (CP) 1

## Figure 6

**Figure 7 :**

**Figure 8**:

Figure 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2833016
- EP 2314669 A
- FR 1201990

**Littérature non-brevet citée dans la description**

- inverse distance weighting. *Lloyd,* 2011, 156
- *Lloyd,* 2011, 74
- Référentiel pédologique. Association française, 2009
- **CARSON, JK ; V GONZALEZ-QUINONES ; DV MURPHY ; C HINZ ; JA. SHAW ; DB GLEESON.** Low Pore Connectivity Increases Bacterial Diversity in Soil. *Appl. Environ. Microbiol.,* 2010, vol. 76 (12), 3936-3942
- **CHO, J-C ; JM TIEDJE.** Biogeography and Degree of Endemicity of Fluorescent Pseudomonas Strains in Soil. *Appl. Environ. Microbiol.,* Décembre 2000, vol. 66 (12), 5448-5456
- **CLAUDE, PP ; L FILLON.** Effet de l'apport d'un inoculum bactérien aux résidus de culture de maïs-grain au sol sur le rendement et la qualité de blés d'hiver panifiables en France. *Agrosol. (depuis - Agrosolutions),* 2004, vol. 15 (1), 23-29
- **COLEMAN, K ; DS JENKINSON.** RothC-26.3, A model for the turnover of carbon in soil : models description and Windows users guide. Rothamsted Research, 2008
- **CURTIS, TP ; WT SLOAN ; JW SCANNELL.** Estimating prokaryotic diversity and its limits. *PNAS,* 06 Août 2002, vol. 99 (16), 10494-10499
- **DUMANSKI, J. ; W.W. PETTAPIECE ; D.F. ACTON ; P.P. CLAUDE.** Application of agro-ecological concepts and hierarchy theory in the design of databases for spatial and temporal characterisation of land and soil. *Geoderma,* 1993, vol. 60, 343-358 343
- **FIERER, N. ; MA BRADFORD ; RB JACKSON.** Toward an ecological classification of soil bacteria. *Ecology,* 2007, vol. 88 (6), 1354-1364
- **FIERER, N. ; MA. BRADFORD ; RB. JACKSON.** Toward An Ecological Classification Of Soil Bacteria. *Ecology,* 2007, vol. 88 (6), 1354-1364
- **FOX, GA ; R METLA.** Soil Property Analysis using Principal Components Analysis, Soil Line, and Regression Models. *Soil Sci. Soc. Am. J.,* 2005, vol. 69, 1782-1788
- **FOX, GA ; GJ SABBAGH.** Estimation of Soil Organic Matter from Red and Near-Infrared Remotely Sensed Data Using a Soil Line Euclidean Distance Technique. *Soil Sci. Soc. Am. J,* 2002, vol. 66, 1922-29
- **FRANKLIN, RB ; AL MILS.** Multi-scale variation in spatial heterogeneity for microbial community structure in an eastern Virginia agricultural field. *FEMS Microbiology Ecology,* 2003, vol. 44, 335-346
- **GAILLARD et al.** Carbon, nitrogen and microbial gradients induced by plant residues decomposing in soil. *Eur. J. Soil Sci.,* 1999, vol. 50, 567-578
- **GAILLARD et al.** Carbon minéralisation in soil adjacaent to plant residues of contrasting biochemical quality. *Soil Biol. Biochem.,* 2003, vol. 35, 93-99
- **GAREY A. FOX ; ROOPA METLA.** Soil Property Analysis using Principal Components Analysis, Soil Line, and Regression Models. *oil Sci. Soc. Am. J,* 2005, vol. 69, 1782-1788
- Base de Données Analyse des Terres. Groupement d'intérêt scientifique sol, 01 Mars 2006
- **GRUNAMPCANN, G. L. ; P. NORMAND.** Microscale Diversity of the Genus Nitrobacter in Soil on the Basis of Analysis of Genes Encoding rRNA. *Appl. Environ. Microbiol.,* Octobre 2000, vol. 66 (10), 4543-4546
- **HALSALL, DM ; AH GIBSON.** Nitrogenase activity in straw-amended wheat belt soils in response to diazotrophe inoculation. *Soil Biology and Biochemistry,* 1991, vol. 23 (10), 987-998
- **HUSAIN et al.** Kinetic Study of a Bacterial Consortium Isolated from Soil Contaminated with Crude Oil. *Australian Journal of Basic and Applied Sciences,* 2011, vol. 5 (6), 925-930
- **JACQUES et al.** Utilisation de consortia fongi - bactériens pour la dégradation in situ de composés poly-aromatiques (PAH). *Biores. Technol.,* 2008, vol. 99 (7), 2637-43
- **JAMAGNE, M.** Grands paysages pédologiques de la France. 2011

- **KADONO, A. et al.** Factors controlling potentially mineralizable and récalcitrant soil organic matter in humid Asia. *Soil. Sci. Plant. Nutr.,* 2009, vol. 55 (2), 243-251
- **LEBART, L ; A MORINEAU ; M. PIRON.** Statistique exploratoire multidimensionnelle. 2000
- **LLOYD, CD.** Local models for spatial analysis. CRC Press, 2011
- **MENDES et al.** Dechipering ring the Rhizosphere Microbiome for Disease-Suppressive Bacteria. *Science,* 2011, vol. 332, 1097
- **MARTINY, JBH ; JA EISEN ; K PENN ; SD ALLISON ; M-C HORNER-DEVINE.** Drivers of bacterial β -diversity depend on spatial scale. *PNAS,* 10 Mai 2011, vol. 108 (19), 7850-7854
- **MCBRATNEY ; ALEX. B. INAKWU ; O.A. ODEH.** Application of fuzzy sets in soil science: fuzzy logic, fuzzy measurements and fuzzy decisions. *Geoderma,* 1997, vol. 77, 85-113
- **MORENO, J ; T. DE LA RUBIA ; A RAMOS-ORMENZANA ; GR VELA.** Growth and nitrogenase activity of Azotobacter vinelandii on soil phenolic acids. *Journal of Applied Bacteriology,* 1990, vol. 69, 328
- **QUINN, JA ; DB MCKAY ; B ENTSCH.** Analysis of the pobA and pobR genes controlling expression of p-hydroxybenzoate hydroxylase in Azotobacter chroococcum. *Gene,* 2001, vol. 264, 77-85
- **RADIANINGTYAS et al.** Characterization of a soil-derived bacterialconsortium degrading 4-chloro-aniline. *Microbiology,* 2003, vol. 149, 3279-3287
- **RAMETTE, ALBAN ; JAMES M. TIEDJE.** Multiscale responses of microbial life to spatial distance and environmental heterogeneity in a patchy ecosystem. *PNAS 104,* 2007, vol. 8, 2761-2766
- **RICHAUME et al.** Évaluation des modifications quantitatives, qualitatives et fonctionnelles induites par la conservation de consortiums bactériens extraits de sols. *Les Actes du BRG,* 2006, vol. 6, 371-389
- **ROPER MM ; RR GAULT ; NA SMITH.** Contribution to the N status of soil by free living nitrogen fixing bacteria in a lucerne stand. *Soil Biol. Biochem.,* 1995, vol. 27 (4/5), 467-471
- **SESSITSCH, A ; A WEILHARTER ; MH. GERZABEK ; H KIRCHMANN ; E KANDELER.** Microbial Population Structures in Soil Particle Size Fractions of a Long-Term Fertilizer Field Experiment. *Appl. Environ. Microbiol.,* 2001, vol. 67 (9), 4215-4224
- **TRAN, L.T. et al.** Application of fuzzy logic-based modeling to improve the performance of the Revised Universal Soil Loss Equation. *CATENA,* 2002, vol. 47 (3), 203-226
- Using fuzzy logic based modelling to improve the performance of the revised universal sol loss equation. **TRAN, L.T. ; M.A. RIDGLEY ; M.A. NEARING ; L. DUCKSTEIN ; R. SUTHERLAND.** Sustaining the Global Farm. Purdue University, 2001
- **TURNER, BL ; BJ CADE-MENUN ; DT WESTERMANN.** Organic Phosphorus Composition and Potential Bioavailability in Semi-Arid Arable Soils of the Western United States. *Soil Sci. Soc. Am. J,* 2003, vol. 67, 1168-1179
- **VAN DER GUCHT ; K, K COTTENIE ; K MUYLAERT VLOEMANS ; N, S COUSIN ; S DECLERCK ; E JEPPESEN ; J-M CONDE-PORCUNA ; K SCHWENK ; G ZWART ; H DEGANS.** The power of species sorting: Local factors drive bacterial community composition over a wide range of spatial scales. *PNAS,* 2007, vol. 104 (51), 20404-20409
- **VOGEL, J. ; P. NORMAND ; J. THIOULOUSE ; X. NESME ; G. L. GRUNAMPCANN.** Relationship between Spatial and Genetic Distance in Agrobacterium spp. in 1 Cubic Centimeter of Soil. *Appl. Environ. Microbiol.,* Mars 2003, vol. 69 (3), 1482-1487
- **ZADEH, L.A.** *Fuzzy sets. Information and Control,* 1965, vol. 8, 338-353
- **ZHU, X. ; B. HUDSON ; J. BURT ; K. LUBICH ; D. SIMONSON.** Soil Mapping Using GIS, Expert Knowledge, and Fuzzy Logic. *Soil Sci. Soc. Am. J.,* 2001, vol. 65, 1463-1472